# EUROPEAN PATENT SPECIFICATION

(11) **EP 4 151 622 B1**
(45) Date of publication and mention of the grant of the patent: **06.05.2026**
(21) Application number: 21804434.5
(22) Date of filing: 28.04.2021
(51) Int. Cl.: C07C 237/04, C07D 211/14, A61K 31/14, A61K 31/40, A61P 17/00, A61P 23/02

(54) **AROMATIC COMPOUND, PREPARATION METHOD THEREFOR AND USE THEREOF IN DRUG**
AROMATISCHE VERBINDUNG, HERSTELLUNGSVERFAHREN DAFÜR UND VERWENDUNG DAVON IN EINEM ARZNEIMITTEL
COMPOSÉ AROMATIQUE, SON PROCÉDÉ DE PRÉPARATION ET SON UTILISATION DANS UN MÉDICAMENT

(30) Priority: 11.05.2020 CN 202010394931
(43) Date of publication of application: 22.03.2023
(73) Proprietor: Sichuan Academy of Medical Sciences Sichuan Provincial People's Hospital, Chengdu, Sichuan 610000 (CN); Sichuan Daozhen Technology Co., Ltd, Chengdu, Sichuan 610041 (CN)
(72) Inventor: TONG, Rongsheng, Chengdu, Sichuan 610000 (CN); SHI, Jianyou, Chengdu, Sichuan 610000 (CN); LIU, Liming, Chengdu, Sichuan 610041 (CN); QIU, Dan, Chengdu, Sichuan 610041 (CN)
(74) Representative: Wilson, Justin Scott
(86) International application number: PCT/CN2021/090726
(87) International publication number: WO 2021/227882

(56) References cited:
- WO-A1-2017/024037
- CN-A- 108 348 774
- CN-A- 110 156 665
- DE-A1- 1 929 223
- US-A- 3 014 046
- US-A- 4 026 708
- RENSHAW R R ET AL: "BASIS FOR THE PHYSIOLOGICAL ACTIVITY OF ONIUM COMPOUNDS XIII Betaine Amides", 1 November 1933 (1933-11-01), pages 183 - 186, XP093177843, Retrieved from the Internet <URL:https://doi.org/10.1016/S0021-9258(20)70813-6>
- SPEZIALE A J ET AL: "Preparation of 2-Substituted Acetamides", no. 78, 1 November 1956 (1956-11-01), pages 5580 - 5584, XP093177852, Retrieved from the Internet <URL:https://pubs.acs.org/doi/abs/10.1021/ja01602a032>
- COLLINS R. F. ET AL: "Chemotherapy of fascioliasis: I.-Derivatives of di-( p -aminophenyl) methane and p -aminophenol", JOURNAL OF THE SCIENCE OF FOOD AND AGRICULTURE, vol. 20, no. 11, 1 November 1969 (1969-11-01), GB, pages 690 - 695, XP093178076, ISSN: 0022-5142, DOI: 10.1002/jsfa.2740201115
- BY R J CLARK ET AL: "DERIVATIVES OF 3: 4-XYLIDINE AND RELATED COMPOUNDS AS INHIBITORS OF INFLUENZA VIRUS: RELATIONSHIPS BE- TWEEN CHEMICAL STRUCTURE AND BIOLOGICAL ACTIVITY", BRITISCH JOURNAL OF PHARMACOLOGY, vol. 13, 1 January 1958 (1958-01-01), pages 424 - 435, XP055155591, DOI: 10.1111/j.1476-5381.1958.tb00233.x
- DATABASE REGISTRY 7 April 2006 (2006-04-07), ANONYMOUS : " 1-Propanaminium, N-[2-[[4-(acetylamino)-2,6-dichlorophenyl]amino]-2- oxoethyl]-N,N-diethyl-, bromide (1:1) (CA INDEX NAME)", XP055867434, retrieved from STN Database accession no. 879638-84-3
- DATABASE REGISTRY 7 April 2006 (2006-04-07), ANONYMOUS : "Ethanaminium, 2-[(4-hydroxyphenyl)amino]-N,N,N-trimethyl-2-oxo-, chloride (1:1) (CA INDEX NAME)", XP093009629, retrieved from STN Database accession no. 879681-57-9

## Description

### FIELD

The present invention relates to an aromatic compound, a production method and use in medicine. The compound can be used in the production of local anesthesia drugs to produce long-term local nerve block effect.

### BACKGROUND

Most of the moderate-to-severe pain caused by surgical wounds will last for 48-72 hours, so postoperative pain is usually managed with systemic opioid analgesic drugs, but the resulting problems such as addiction, constipation, and respiratory depression plague patients. How to ensure safe and effective postoperative analgesia while reducing the use of opioids has become a major clinical problem that needs to be solved.

Clinically, local anesthetic drugs can be used to control pain in some surgery with minor wounds, thereby avoiding or reducing the use of systemic opioid anesthetic drugs. Local anesthesia is a common clinical medical operation, which refers to the application of local anesthetic drugs to parts of the body when the patient is awake, so that the sensory nerve conduction function of a certain part of the body is temporarily blocked to produce analgesic effect, and the motor nerve conduction remains intact or is blocked of varying degrees simultaneously. This block should be fully reversible. The advantages of local anesthesia lie in its simplicity, safety, patient awareness, few complications and little impact on the patient's physiological function. Local anesthesia is achieved through the use of local anesthetic drugs, and the duration and intensity of the action of local anesthetic drugs determine the duration and analgesic intensity of local anesthesia. Due to the continuous diffusion of the drug from the administration site, the local concentration of the drug decreases. All local anesthetic drugs currently used in clinical use produce a duration of local anesthesia within 6 hours after a single administration. Even if the local anesthetic drugs are used in combination with drugs such as vasoconstrictor dexamethasone, and the diffusion loss of local anesthetic drugs is reduced at the administration site, the duration of local anesthesia can still not exceed 10 hours (Kyle Robert Kirkham et al, 2018, Anesthesia & Analgesia. 126(1):270-279). In 2011, a liposomal sustained-release preparation containing bupivacaine was launched in the United States. Although the preparation was alleged to produce a 72-hour local anesthetic effect, it has been reported to demonstrate its unstable dose-drug dependence (Christopher Wahal, Indian J Anaesth. 2018; 62(2): 94-102.) and limited analgesic effect (Malik O et al, J Anaesthesiol Clin Pharmacol. 2017;33(2):151-156), suggesting that the preparation cannot cope with pain independently for 48 to 72 hours after surgery and can only reduce the use of systemic opioid analgesic drugs. In some subsequent clinical trials, liposome of bupivacaine was shown to provide only 24 hours of postoperative analgesic effect, but there was no statistical difference in pain intensity between the administration group and the placebo group during the 24-72 hour period (Uskova A et al, Curr Opin Anaesthesiol. 2015;28(5):593-7.). It can be seen that if local anesthetic drugs have sufficient analgesic intensity and action duration, it is expected to greatly reduce the use of opioids and avoid or reduce various systemic side effects caused by opioids.

Another challenge with long-acting local anesthetic drugs is drug safety, including both local and systemic safety. Local safety mainly refers to whether the degree of tissue damage (especially nerve damage) at the administration site is within a clinically acceptable range. The systemic safety refers to whether the drug will have a toxic effect on the heart if it diffuses or is mistakenly injected into the systemic blood circulation, and whether it will have a toxic effect on the central nervous system if it passes through the blood-brain barrier. All local anesthetic drugs currently used clinically have systemic toxicity, and if they are administrated in a too large dosage or injected into the blood by mistake, they can cause arrhythmia, abnormal central nervous activity and even death (Wadlund DL. AORN J.2017 Nov;106(5):367 -377).

In order to obtain longer-acting local anesthetic drugs, some improved molecules for traditional local anesthetic drugs that do not have lasting efficacy due to diffusion have appeared one after another, such as QX-314 and QX-314-OH, which are the quaternary ammonium salt derivatives of the traditional local anesthetic drug lidocaine, having a similar structure to lidocaine. However, the molecules of the QX-314 and QX-314-OH have a permanent positive charge, which slows the diffusion of the molecules, enabling them to produce local anesthesia for a significantly longer duration than lidocaine (2 hours VS 6-8hours). It is worth noting that although the introduction of cations prolongs the duration of local anesthesia of the molecules, the ability of the molecules to diffuse from the injection site to the nerve cells is not good, and the time of the onset of action of QX-314 and QX-314-OH is also significantly delayed, which needs 20-30 minutes to take effect after injection (Anesthesiology, 2009, 111:122-6). In addition, in order to fully exert the functions of the above charged molecules, certain ion channel openers are required to deliver the above molecules into the cells to exert their functions (CN201680057337.3).

Other surface-active quaternary ammonium derivatives of lidocaine that can self-assemble to form sustained-release materials were subsequently reported. These monomeric molecules can produce long-acting (24-90 hours) local anesthetic effects after assembly in solution (Chinese invention application: 201510423735.X), but such molecules can cause moderate to severe nerve damage to local nerves (Chinese invention application: 201810104560.X). Subsequently, some new cationic long-acting local anesthetic drugs were reported (201910100568.3, 201910101369.4). The doses of these compounds in the rat sciatic nerve block model were relatively high, indicating that their titer is low, and the overall safety of these molecules is unclear.

It can be seen that long-acting local anesthetic drugs which have rapid onset of action at a single administration, high titer, small local damage, and good overall safety after administration still need to be further researched and developed.

### SUMMARY

In view of the deficiencies of current long-acting local anesthetic drugs or drug-like molecules, the present invention discloses a new class of cationic aromatic compounds, and provides a production method thereof and use of such compounds in medicine.

The amidated compound disclosed herein has the structure as shown in formula (I): 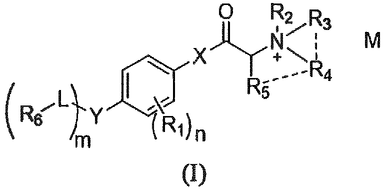
wherein, R1 is optionally selected from the group consisting of C1-5 hydrocarbyl, alkoxyl, hydroxyl, amino or substituted amino, carboxyl, halogen, cyano, alkoxycarbonyl, etc.; R2 is selected from the group consisting of C1-8 hydrocarbyl; R3 is selected from the group consisting of C1-8 hydrocarbyl and hydrocarbylene; R4 is selected from the group consisting of C1-8 hydrocarbyl and hydrocarbylene; R5 is selected from the group consisting of H, C1-8 hydrocarbyl and hydrocarbylene; R3 and R4 can also be directly connected by a chemical bond to form a ring with quaternary ammonium N atom; R4 and R5 can also be directly connected by a chemical bond to form a ring with quaternary ammonium N atom; X is O or HN or N substituted with alkyl; Y is O or OH or NH2 or N substituted with alkyl; L is carbonyl or absent; m=0 or 1; n is an integer from 0 to 4;
R6 is C1-18 hydrocarbyl or the following specified structure:

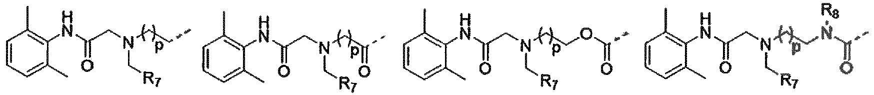

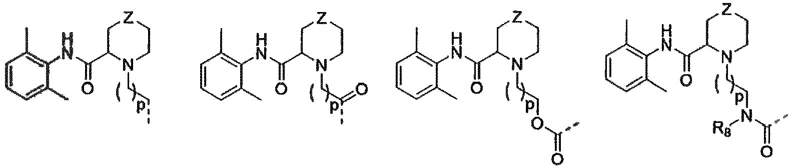

In the above specified structure, p is an integer from 1 to 16, R7 is C1-7 hydrocarbyl, R8 is H or C1-8 hydrocarbyl, Z is methylene or O; any carbon atom in the skeleton of R1-R7 can be replaced by O, S, sulfone group, sulfoxide group, and N atom; the skeleton of R1-R7 can have one or more substituents, including: halogen, nitro, cyano, carboxyl, ester group, and hydroxyl; M is a pharmaceutically acceptable anion, and if the molecule (I) has an internal salt structure, M may be absent.

Preferred compounds meeting the above structural features include, but are not limited to:

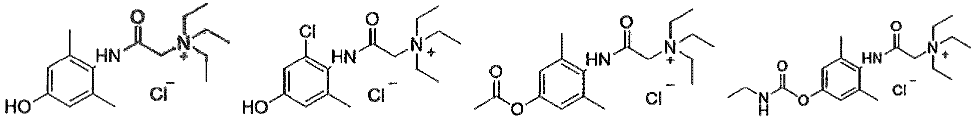

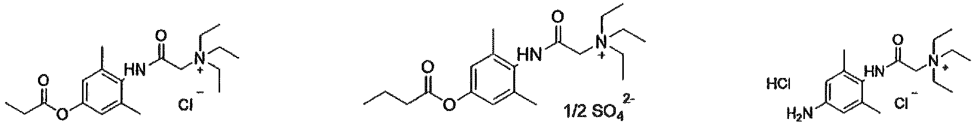

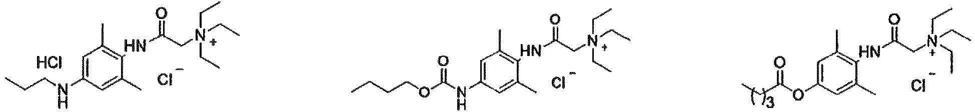

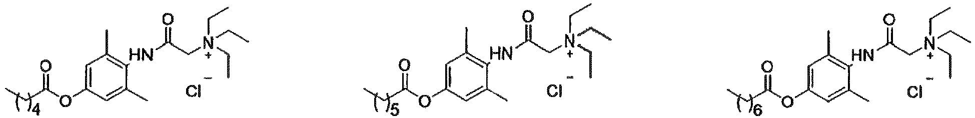

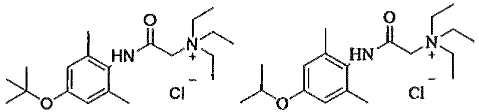

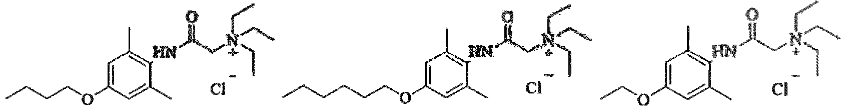

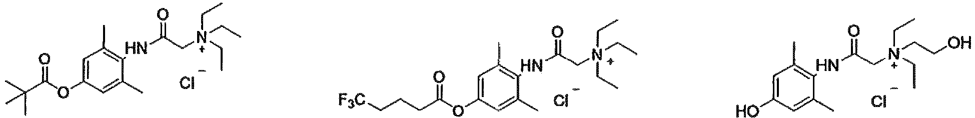

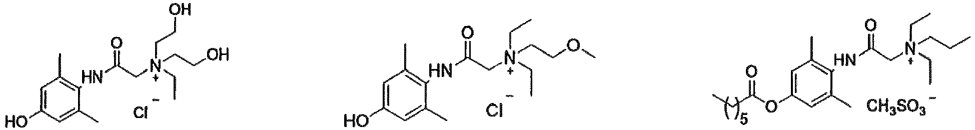

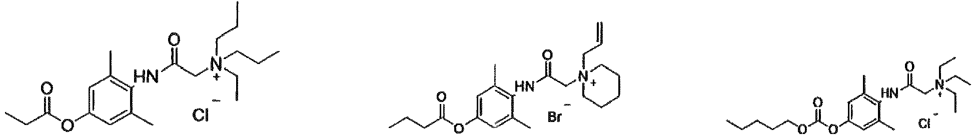

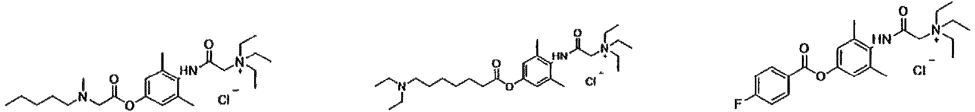

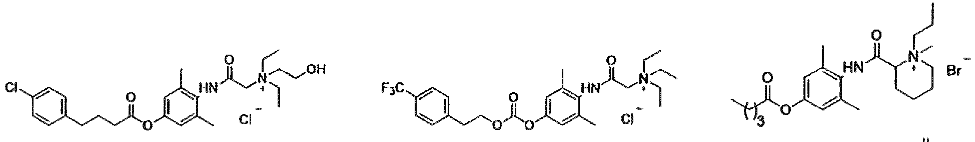

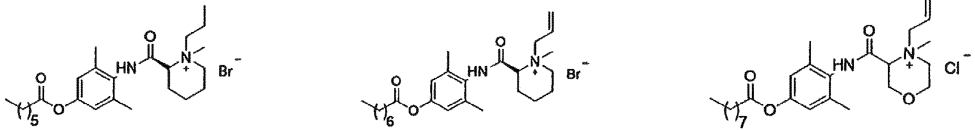

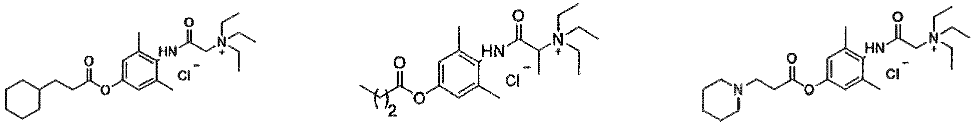

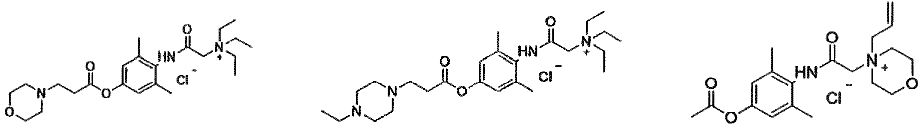

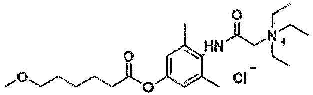

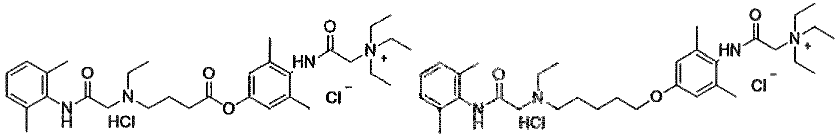

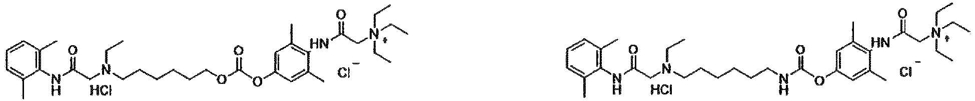

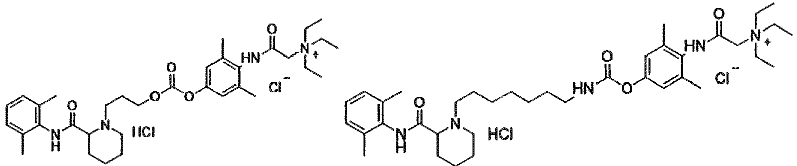

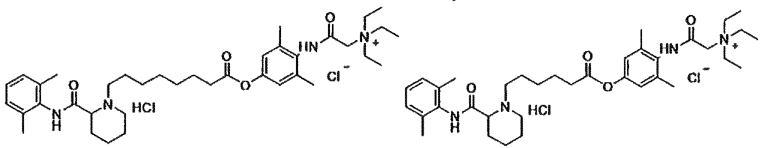

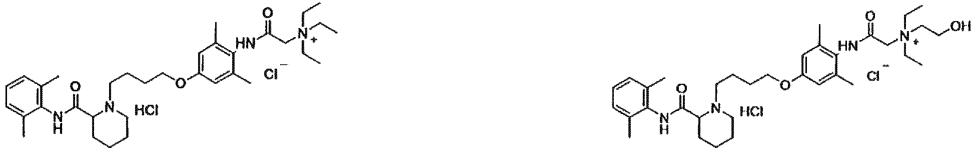

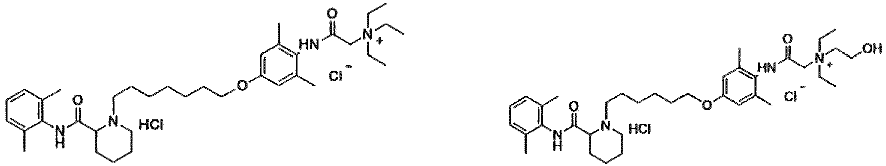

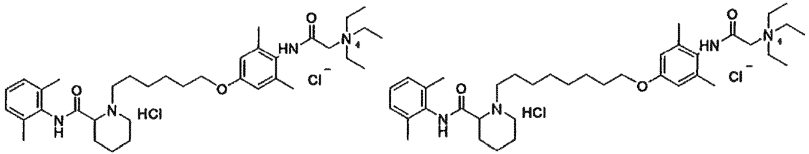

Such compounds are usually obtained by synthesizing the molecules containing A and B structural fragments respectively, and then bonding the A and B fragments with a covalent bond or via a linker: 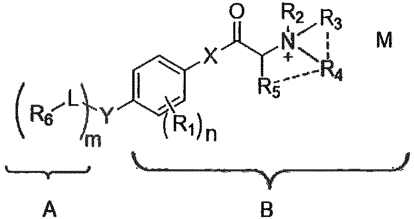
when the linker is 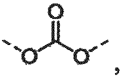 the general synthetic route for the target is: 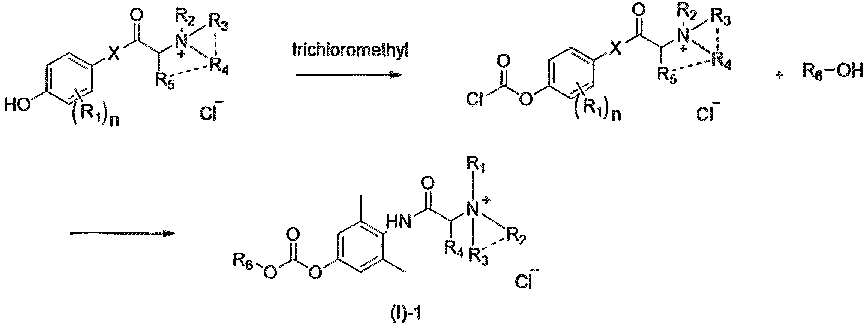
when the linker is 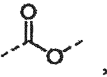 , the general synthetic route for the target is: 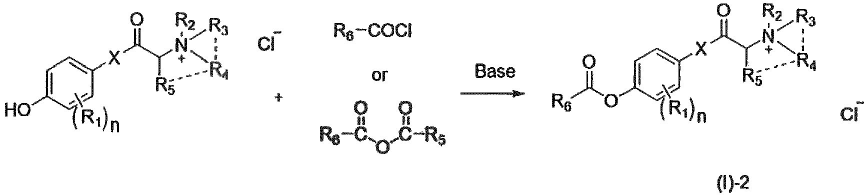
when the linker is target is: 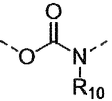 (R10 is H or alkyl), the general synthetic route for the 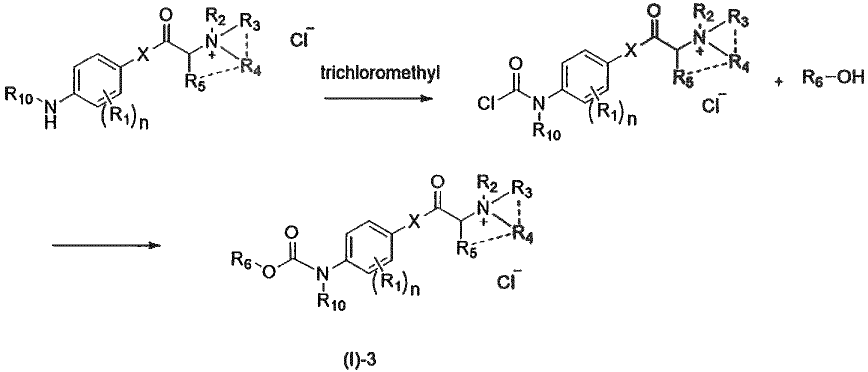
when the linker is 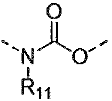 (R11 is H or alkyl), the general synthetic route for the target is: 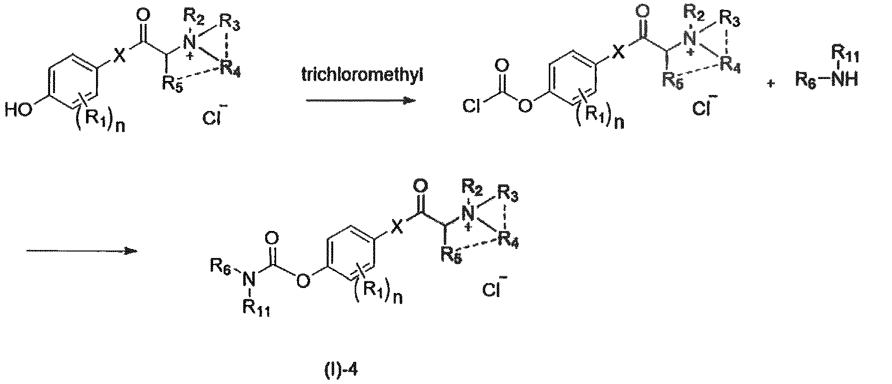
when the linker is 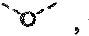 the general synthetic route for the target is (X is halogen or sulfonate):

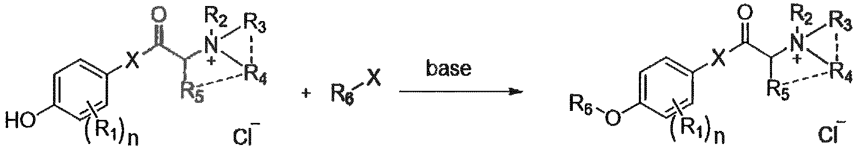

In the two experiments of sciatic nerve block and subcutaneous infiltration anesthesia in rats, the compounds of the present invention can produce rapid, strong and long-term blockade (24-96 hours) on local nerves at relatively low concentrations (0.2-0.5%) alone. It has good safety to tissues within a wide concentration range, and can achieve long-acting local anesthesia while slightly damaging nerve and muscle tissue. Within the therapeutic dose range, the compounds disclosed in the present invention did not exhibit clinically unacceptable damage such as nerve necrosis and demyelination, and meanwhile, there is no significant difference in inflammation score between the compounds of the present invention and levobupivacaine with a clinical concentration of 0.75%. It shows that the compounds disclosed in the present invention have good local safety. In addition, the compounds disclosed in the present invention also have obvious advantages in terms of systemic toxicity, which is manifested in the fact that the intravenous injection of the compounds in an effective dose for local anesthesia did not cause death of animals, while the injection of 0.75% of levobupivaca through the tail vein in an effective dose for local anesthesia can cause the death of most animals. The above results indicate that the molecules disclosed in the present invention are generally safe, and there is no concern of serious acute toxicity after the compounds are mistakenly injected into blood. More unexpectedly, when the compounds of the present invention are used in combination with common clinical local anesthetic drugs such as lidocaine, bupivacaine or tetracaine, the dose required for their onset of action is further reduced, showing a good synergistic effect of the compounds with these drugs, and this feature enhances the safety of the compounds of the present invention. Finally, in the sciatic nerve block experiment on the compounds of the present invention, the changes in the motor ability of animals were not observed, showing the advantage of selective sensory block.

The unexpected safety improvement and high titer of the compounds of the present invention greatly improve the potential of such drugs for clinical postoperative analgesia. The compounds disclosed in the present invention, pharmaceutically acceptable salts, isotopic molecules, solvates, single crystal forms or co-crystals, optical isomers, and preparations (including sustained-release preparations), pharmaceutical kits and compound recipe comprising the above substances can be used in the production of long-acting local anesthetic drugs.

### DETAILED DESCRIPTION

The content and advantages of the present invention are described below with reference to the examples. Wherein, Examples that do not form part of the invention are reference examples which represent background art that is useful for understanding the invention.

### Example 1

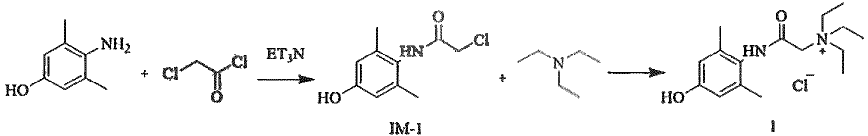

13.7 g of 4-amino-3,5-dimethylphenol and 40 g of triethylamine were dissolved in 150 mL of dichloromethane, and 11.3 g of chloroacetyl chloride was slowly added dropwise to the mixture under an ice bath. After the dropping was completed, the mixture was stirred to react for 2 hours, then the reacted mixture was evaporated off solvent to dryness under reduced pressure, and 150 mL of ethyl acetate was added to the residue. 50 mL of dilute hydrochloric acid was used to wash the organic layer, and then the organic layer was separated out. The separated organic layer was evaporated off ethyl acetate to dryness under reduced pressure to obtain brown-black oil, which was then purified by column chromatography (petroleum ether/ethyl acetate=3/1) to obtain 4.89 g of a yellow solid powder (IM-1) with a yield of 22.9%.

300 mg of IM-1 was dissolved in 3 mL of anhydrous ethanol, and the mixture was added with 2 mL of triethylamine. The obtained mixture was stirred in a sealed tube at an external temperature of 95 °C to react for 3 hours to precipitate a white solid. The obtained mixture was cooled, and added with 5 mL of ethanol. 2 drops of concentrated hydrochloric acid was used to adjust the pH to below 3. The mixture was evaporated off ethanol to dryness under reduced pressure to obtain a grey-white solid powder, which was then purified by column chromatography (dichloromethane/methanol=10/1) to obtain 122 mg of white powder, namely compound 1, with a yield of 27.6%.

1HNMR d-DMSO 400Hz: 1.29 (9H, t, J=8Hz), 2.07 (6H, s), 3.51 (6H, q, J=8Hz), 4.33 (2H, s), 6.49 (2H, s), 9.32 (1H, s), 10.21 (1H, s).

### Example 2

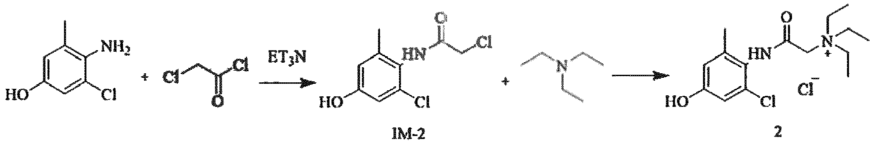

15.7 g of 4-amino-3-chloro-5-methylphenol and 40 g of triethylamine were dissolved in 150 mL of dichloromethane, and 11.3 g of chloroacetyl chloride was slowly added dropwise to the mixture under an ice bath. After the dropping was completed, the mixture was stirred to react for 2 hours, then the reacted mixture was evaporated off solvent to dryness under reduced pressure, and 150 mL of ethyl acetate was added to the residue. 50 mL of dilute hydrochloric acid was used to wash the organic layer and then the organic layer was separated out. The separated organic layer was evaporated off ethyl acetate to dryness under reduced pressure to obtain brown-black oil, which was then purified by column chromatography (petroleum ether/ethyl acetate=3/1) to obtain 3.22 g of a yellow solid powder (IM-2) with a yield of 13.8%.

300 mg of IM-2 was dissolved in 3 mL of anhydrous ethanol, and the mixture was added with 2 mL of triethylamine. The obtained mixture was stirred in a sealed tube at an external temperature of 95 °C to react for 3 hours to precipitate a white solid. The obtained mixture was cooled, and added with 5 mL of ethanol. 2 drops of concentrated hydrochloric acid was used to adjust the pH to below 3, and then the mixture was evaporated off ethanol to dryness under reduced pressure to obtain a grey-white solid powder, which was then purified by column chromatography (dichloromethane/methanol=10/1) to obtain 109 mg of a white powder, namely compound 2, with a yield of 25.4%.

1HNMR d-DMSO 400Hz: 1.26 (9H, t, J=8Hz), 2.14 (3H, s), 3.48 (6H, q, J=8Hz), 4.31 (2H, s), 6.52 (1H, s), 7.18 (1H, s), 9.21 (1H, s), 10.18 (1H, s).

### Example 3

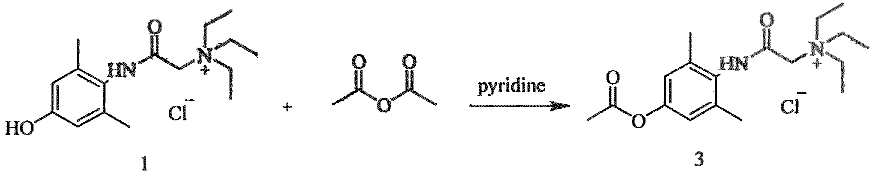

600 mg of compound 1 was mixed with 1 g of acetic anhydride, and 3 mL of pyridine was added to the mixture. The obtained mixture was stirred in a sealed tube at an external temperature of 90 °C for 18 hours. Silica gel was added to the mixture to mix sample, then the resulting mixture was evaporated to dryness under reduced pressure, and the residue was subjected to column chromatography (dichloromethane/methanol=20/1) to obtain yellow oil, which was then recrystallized with ethyl acetate to precipitate a white solid. The obtained mixture was filtered, and the obtained solid was dried at 50°C to obtain 350 mg of white solid powder, namely compound 3, with a yield of 51.5%.

1HNMR d-DMSO 400Hz: 1.31 (9H, t, J=8Hz), 2.17 (6H, s), 2.25 (3H, s), 3.53 (6H, q, J=8Hz), 4.42 (2H, s), 6.88 (2H, s), 10.66 (1H, s).

### Example 4

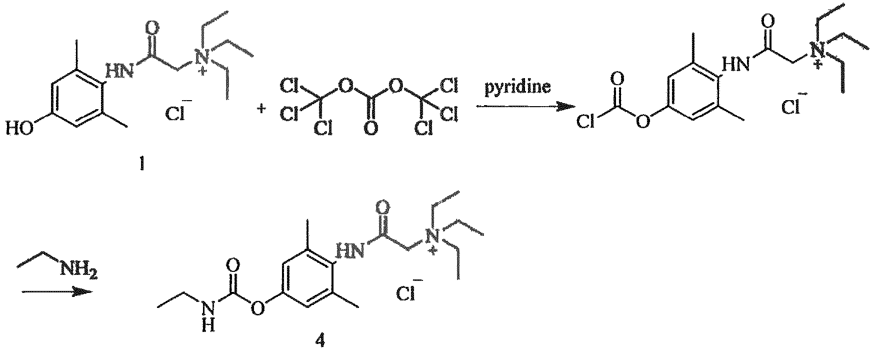

500 mg of compound 1 was dissolved in 30 mL of dichloromethane, and 200 mg of triphosgene was added to the mixture. The obtained mixture was cooled to 0-5°C, and slowly added dropwise with 3 mL of pyridine. The resulting mixture was removed from the ice bath, stirred for 2 hours, then cooled down to 0-5 °C, and slowly added with a solution of 50% ethylamine in 1.5 mL of ethanol. The obtained mixture was stirred for 1 hour, removed from the ice bath, stirred at room temperature for 2 hours, and added dropwise with dilute hydrochloric acid to adjust the pH to 3. The reaction solution was then evaporated off solvent to dryness under reduced pressure, and the residue was subjected to column chromatography (dichloromethane/methanol=10/1) to obtain 389 mg of white powder, namely compound 4, with a yield of 63.5%.

1HNMR d-DMSO 400Hz: 1.06 (3H, t, J=8 Hz), 1.29 (9H, t, J=8Hz), 2.07 (6H, s), 3.05 (2H, m), 3.51 (6H, q, J =8Hz), 4.33 (2H, s), 6.49 (2H, s), 6.78 (1H, s), 10.21 (1H, s).

### Example 5

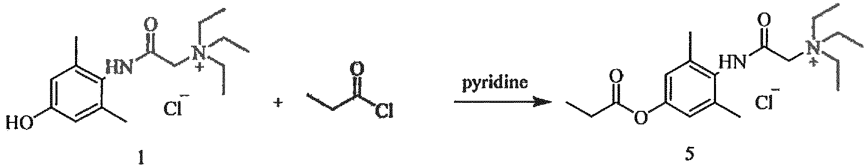

600 mg of compound 1 was mixed with 3 mL of pyridine, and 1 mL of propionyl chloride was added to the mixture. The obtained mixture was stirred at an external temperature of 40 °C for 5 hours. Silica gel was added to the mixture to mix sample, then the resulting mixture was evaporated to dryness under reduced pressure, and the residue was subjected to column chromatography (dichloromethane/methanol=20/1) to obtain yellow oil, which was then recrystallized with ethyl acetate to precipitate a white solid. The obtained mixture was filtered, and the obtained solid was dried at 50°C to obtain 110 mg of white solid powder, namely compound 5, with a yield of 15.6%.

1HNMR d-DMSO 400Hz: 1.13 (3H, t, J=8Hz), 1.29 (9H, t, J=8Hz), 2.17 (6H, s), 2.51-2.61 (2H, m), 3.53 (6H, q, J=8Hz), 4.44(2H, s), 6.88 (2H, s), 10.72 (1H, s).

### Example 6

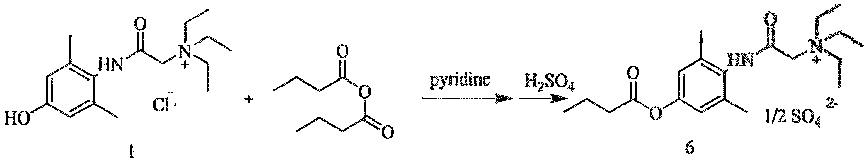

600 mg of compound 1 was mixed with 3 mL of pyridine, and 1 g of n-butyric anhydride was added to the mixture. The obtained mixture was stirred in a sealed tube at an external temperature of 90 °C for 18 hours. 0.5 mol/L sulfuric acid ethanol solution was used to adjust the pH of the reacted mixture to below 3. The mixture was evaporated off solvent to dryness under reduced pressure, and the residue was subjected to column chromatography (dichloromethane/methanol=20/1) to obtain yellow oil, which was then recrystallized with ethyl acetate to precipitate a grey-white solid. The obtained mixture was filtered, and the obtained solid was dried at 50°C to obtain 378 mg of white solid powder, namely compound 6, with a yield of 44.5%.

1HNMR d-DMSO 400Hz: 0.97 (3H, t, J=8Hz), 1.31 (9H, t, J=8Hz), 1.61-1.70 (2H, m), 2.17 (6H, s), 2.49-2.56 (2H, m), 3.53 (6H, q, J=8Hz), 4.41 (2H, s), 6.87 (2H, s), 10.62 (1H, s).

### Example 7

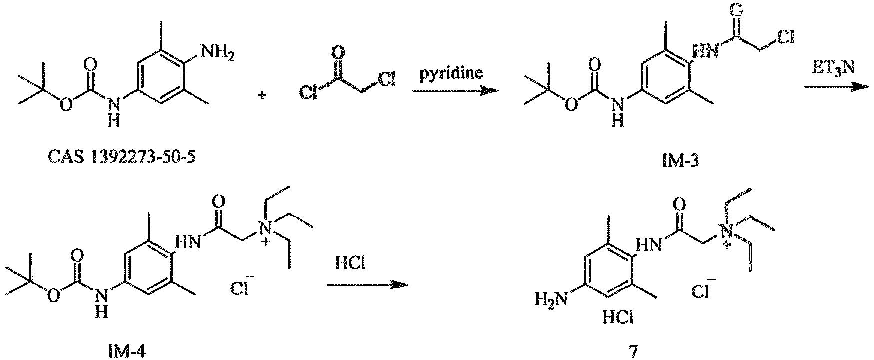

2.36 g of N-boc-3,5-dimethyl-4-aminoaniline (CAS: 1392273-50-5) was dissolved in 30 mL of dichloromethane, the mixture was added with 5 g of pyridine, and slowly added dropwise with 1.3 g of chloroacetyl chloride under an ice bath. After the dropping was completed, the mixture was removed from the ice bath, stirred at room temperature for 2 hours, then evaporated off solvent to dryness under reduced pressure, and 100 mL of ethyl acetate was added to dissolve the residue. The ethyl acetate layer was washed twice with 50 mL of 3N hydrochloric acid for each time, and washed once with water. The organic layer was separated out, and evaporated off the ethyl acetate to dryness under reduced pressure. 50 mL of acetonitrile and 4 g of triethylamine were added to the residue, and the obtained mixture was heated to 65°C to react overnight. The reacted mixture was evaporated off acetonitrile to dryness under reduced pressure, and 50 mL of dichloromethane was added to dissolve the residue. The mixture was introduced with dry hydrogen chloride gas for 30 minutes, and the obtained reaction solution was stirred at room temperature for 3 hours. The reacted solution was evaporated off solvent to dryness under reduced pressure, and the residue was subjected to column chromatography (dichloromethane/methanol = 20/1) to obtain 0.79 g of white powder, namely compound 7, with a yield of 22.6%.

1HNMR d-DMSO 400Hz: 1.27 (9H, t, J=8Hz), 2.05 (6H, s), 3.50 (6H, q, J=8Hz), 4.32 (2H, s), 6.78 (2H, s), 8.32 (3H, s), 10.20 (1H, s).

### Example 8

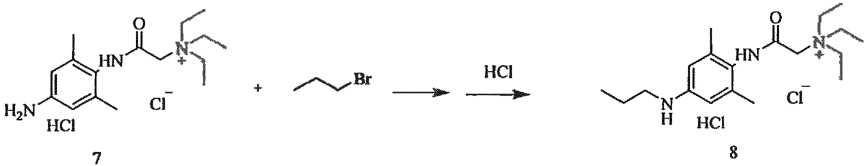

3.5 g of compound 7 was dissolved in 50 mL of acetonitrile, and 1.5 g of anhydrous sodium carbonate was added to the mixture. The obtained mixture was stirred at room temperature for 2 hours and then added with 1.2 g of bromopropane. The resulting mixture was stirred under nitrogen protection in a sealed tube at 75°C overnight. The obtained mixture was cooled and filtered, and the filtrate was introduced with hydrogen chloride gas for 10 minutes, and then evaporated off solvent to dryness under reduced pressure. The residue was subjected to column chromatography (dichloromethane/methanol=20/1) to obtain 1.39 g of white powder, namely compound 8, with a yield of 35.5 %.

1HNMR d-DMSO 400Hz: 0.89 (3H, t, J=8Hz), 1.26 (9H, t, J=8Hz), 1.53-1.58 (2H, m), 2.07 (6H, s), 3.32 (2H, m), 3.51 (6H, q, J=8Hz), 4.31 (2H, s), 6.72 (2H, s), 8.32 (2H, s), 10.13 (1H, s).

### Example 9

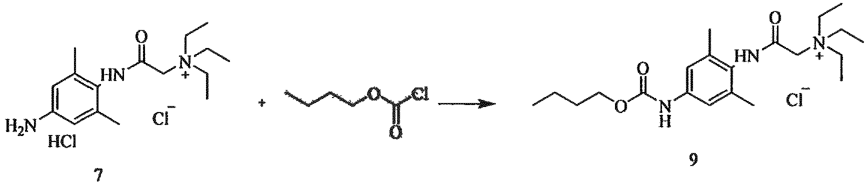

3.5 g of compound 7 was dissolved in 50 mL of acetonitrile, and 1.5 g of anhydrous sodium carbonate was added to the mixture. The obtained mixture was stirred at room temperature for 2 hours and then added dropwise with 1.5 g of butyl chloroformate (CAS: 592-34-7), and the resulting mixture was reacted overnight at room temperature. The reacted mixture was filtered, the filtrate was evaporated off solvent to dryness under reduced pressure, and the residue was subjected to column chromatography (dichloromethane/methanol=20/1) to obtain 2.7 g of white powder, namely compound 9, with a yield of 65.2%.

1HNMR d-DMSO 400Hz: 0.91(3H, t, J=8Hz), 1.24-1.28 (11H, m), 1.43-1.48(2H,m), 2.09 (6H, s), 3.50 (6H, q, J= 8Hz), 3.89-3.93(2H, m), 4.30 (2H, s), 6.75 (2H, s), 8.37 (2H, s), 10.43 (1H, s).

### Example 10

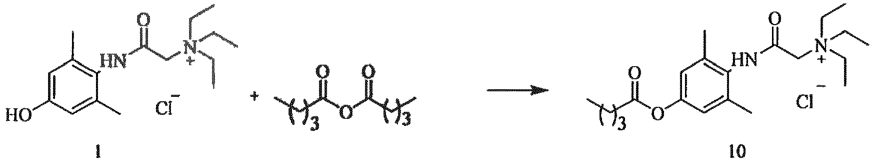

315 mg of compound 1 was mixed with 1 mL of n-valeric anhydride, and 3 mL of pyridine was added to the mixture. The obtained mixture was stirred in a sealed tube at 90 °C for 8 hours, and then evaporated off pyridine to dryness under reduced pressure, and ethyl acetate was added to the residue to precipitate a powder solid. The mixture was filtered to obtain a white solid powder, which was then recrystallized twice with ethyl acetate/ethanol to obtain 198 mg of white solid powder, namely compound 10, with a yield of 49.6%.

1HNMR d-DMSO 400Hz: 0.92 (3H, t, J=8Hz), 1.29-1.39(11H, m), 1.60-1.62(2H, m), 2.17(6H,s), 2.5-2.57(4H, m), 3.53(6H, q, J=8Hz), 4.4 (2H, s), 6.87 (2H, s), 10.59(1H, s).

### Example 11

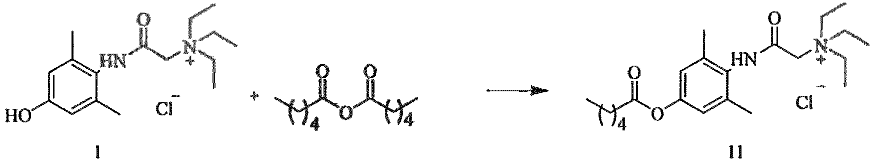

315 mg of compound 1 was mixed with 1 mL of n-hexanoic anhydride, and 3 mL of pyridine was added to the mixture. The obtained mixture was stirred in a sealed tube at 90 °C for 8 hours, and then evaporated off pyridine to dryness under reduced pressure, and ethyl acetate was added to the residue to precipitate a powder solid. The mixture was filtered to obtain a white solid powder, which was then recrystallized twice with ethyl acetate/ethanol to obtain 157 mg of white solid powder, namely compound 11, with a yield of 38%.

1HNMR d-DMSO 400Hz: 0.88-0.91(3H,m), 1.27-1.37(13H,m), 1.60-1.67(2H,m), 2.18(6H,s), 2.49-2.57(2H,m), 3.34 -3.55(6H, m), 4.46(2H, s), 6.86 (2H, s), 10.82 (1H, s).

### Example 12

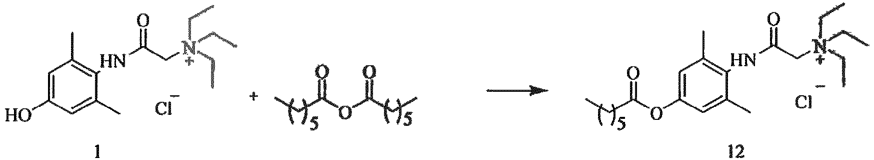

315 mg of compound 1 was mixed with 1 mL of n-heptanoic anhydride, and 3 mL of pyridine was added to the mixture. The obtained mixture was stirred in a sealed tube at 90 °C for 8 hours, and then evaporated off pyridine to dryness under reduced pressure, and ethyl acetate was added to the residue to precipitate a powder solid. The mixture was filtered to obtain a white solid powder, which was then recrystallized twice with ethyl acetate/ethanol to obtain 141 mg of white solid powder, namely compound 12, with a yield of 33%.

1HNMR d-DMSO 400Hz: 0.86-0.90 (3H, m), 1.27-1.38(15H, m), 1.49-1.64 (2H, m), 2.17(6H, s), 2.55(2H, t, J=8Hz), 3.50-3.55(6H, m), 4.42(2H,s), 6.86(2H,s), 10.65(1H,s).

### Example 13

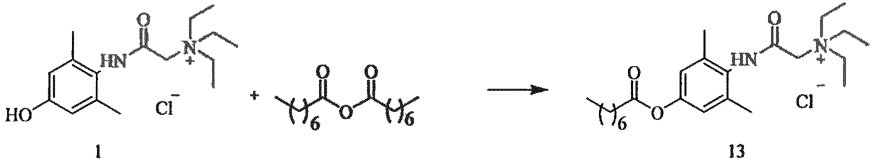

315 mg of compound 1 was mixed with 1 mL of n-octanoic anhydride, and 3 mL of pyridine was added to the mixture. The obtained mixture was stirred in a sealed tube at 90 °C for 8 hours, and then evaporated off pyridine to dryness under reduced pressure, and ethyl acetate was added to the residue to precipitate a powder solid. The mixture was filtered to obtain a white solid powder, which was then recrystallized twice with ethyl acetate/ethanol to obtain 121 mg of white solid powder, namely compound 13, with a yield of 27.4%.

1HNMR d-DMSO 400Hz: 0.86-0.89 (3H, m), 1.29-1.33 (17H, m), 1.59-1.65 (2H, m), 2.17 (6H, s), 2.50-2.56 (2H, m), 3.50 -3.55(6H, m), 4.43 (2H, s), 6.86 (2H, s), 10.68 (1H, s).

### Example 14

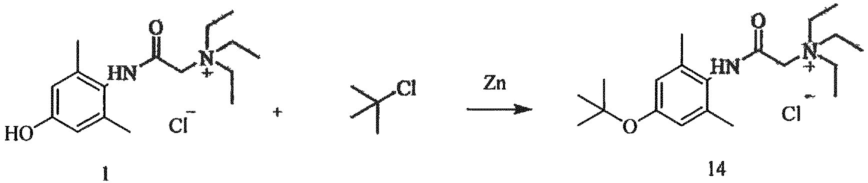

315 mg of compound 1 and 700 mg of zinc powder were mixed and stirred with 30 mL of toluene, and 95 mg of tert-butyl chloride was added to the mixture. The obtained mixture was stirred at room temperature for 2 hours, and evaporated off solvent to dryness under reduced pressure. 30 mL of absolute ethanol was added to the residue. The resulting mixture was filtered through celite, then the filtrate was evaporated to dryness under reduced pressure, and the residue was subjected to column chromatography (dichloromethane/methanol=20/1) to obtain 109 mg of white solid powder, namely compound 14, with a yield of 29.4%.

1HNMR d-DMSO 400Hz: 1.27 (9H, t, J=8Hz), 1.49 (9H, s), 2.05 (6H, s), 3.50 (6H, q, J=8Hz), 4.31 (2H, s), 6.67 (2H, s), 10.21 (1H, s).

### Example 15

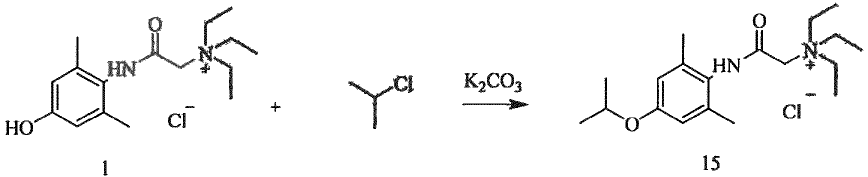

315 mg of compound 1 and 100 mg of 2-chloropropane were mixed in 3 mL of acetonitrile, and 500 mg of anhydrous potassium carbonate and 5 mg of potassium iodide were added to the mixture. The obtained mixture was stirred in a sealed tube at 70 °C overnight. The reacted mixture was cooled and filtered, then the filtrate was evaporated off solvent to dryness under reduced pressure, and the residue was subjected to column chromatography (dichloromethane/methanol=20/1) to obtain 95 mg of white powder, namely compound 15, with a yield of 26.6%.

1HNMR d-DMSO 400Hz: 1.23-1.29 (15H, m), 2.04 (6H, s), 3.51 (6H, q, J=8Hz), 4.30 (2H, s), 4.69-4.73 (1H, m), 6.65 (2H, s), 10.29 (1H, s).

### Example 16

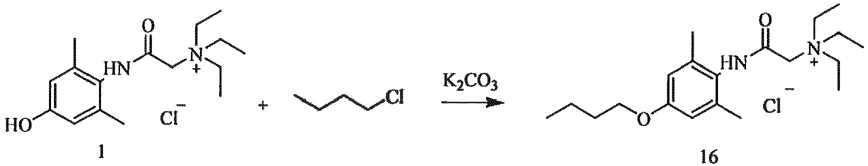

315 mg of compound 1 and 110 mg of 1-chlorobutane were mixed in 3 mL of acetonitrile, and 500 mg of anhydrous potassium carbonate and 5 mg of potassium iodide were added to the mixture. The obtained mixture was stirred in a sealed tube at 70 °C overnight. The reacted mixture was cooled and filtered, then the filtrate was evaporated off solvent to dryness under reduced pressure, and the residue was subjected to column chromatography (dichloromethane/methanol=20/1) to obtain 81 mg of white powder, namely compound 16, with a yield of 21.8%.

1HNMR d-DMSO 400Hz: 0.91-0.94(3H, m), 1.26 (9H, t, J=8Hz), 1.43-1.46(2H, m), 1.68-1.71(2H, m), 2.06 (6H, s), 3.52 (6H, q, J=8Hz), 4.01-4.04 (2H, m), 4.30 (2H, s), 6.63(2H, s), 10.21 (1H, s).

### Example 17

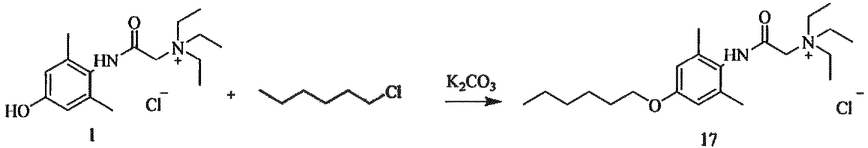

315 mg of compound 1 and 125 mg of 1-chlorohexane were mixed in 3 mL of acetonitrile, and 500 mg of anhydrous potassium carbonate and 5 mg of potassium iodide were added to the mixture. The obtained mixture was stirred in a sealed tube at 70 °C overnight. The reacted mixture was cooled and filtered, then the filtrate was evaporated off solvent to dryness under reduced pressure, and the residue was subjected to column chromatography (dichloromethane/methanol=20/1) to obtain 53 mg of white powder, namely compound 17, with a yield of 13.3%.

1HNMR d-DMSO 400Hz: 0.88-0.90 (3H, m), 1.27 (9H, t, J=8Hz), 1.35-1.45(4H, m), 1.79-1.82(2H, m), 2.09 (6H, s), 3.51 (6H, q, J=8Hz), 4.02-4.05 (2H, m), 4.28 (2H, s), 6.61(2H, s), 10.29(1H, s).

### Example 18

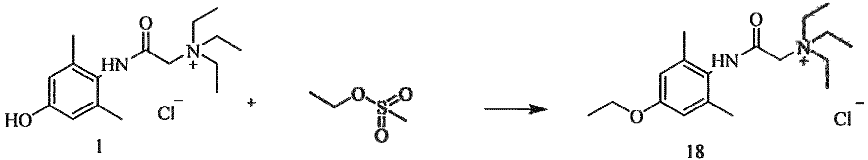

315 mg of compound 1 and 130 mg of ethyl methanesulfonate were mixed in 10 mL of acetonitrile, and 800 mg of triethylamine was added to the mixture. The obtained mixture was stirred in a sealed tube at 70 °C overnight. The reacted mixture was cooled and filtered, then the filtrate was evaporated off solvent to dryness under reduced pressure, and the residue was subjected to column chromatography (dichloromethane/methanol=20/1) to obtain 213 mg of white powder, namely compound 17, with a yield of 62.1%.

1HNMR d-DMSO 400Hz: 1.25-1.35 (12H, m), 2.06 (6H, s), 3.50 (6H, q, J=8Hz), 4.01-4.05 (2H, m), 4.29 (2H, s), 6.60 (2H, s), 10.23 (1H, s).

### Example 19

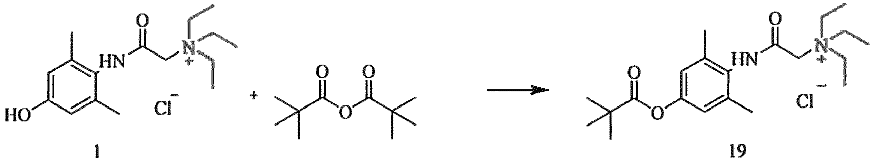

315 mg of compound 1 was mixed with 1 mL of trimethylacetic anhydride, and 3 mL of pyridine was added to the mixture. The obtained mixture was stirred in a sealed tube at 90 °C for 8 hours, then the obtained mixture was evaporated off pyridine to dryness under reduced pressure, and ethyl acetate was added to the residue to precipitate a powder solid. The mixture was filtered to obtain a white solid powder, which was then recrystallized twice with ethyl acetate/ethanol to obtain 78 mg of white solid powder, namely compound 19, with a yield of 19.5%.

1HNMR d-DMSO 400Hz: 1.29-1.32 (18H, m), 2.17 (6H, s), 3.51 (6H, q, J=8Hz), 4.37 (2H, s), 6.86 (2H, s), 10.46 (1H), s).

### Example 20

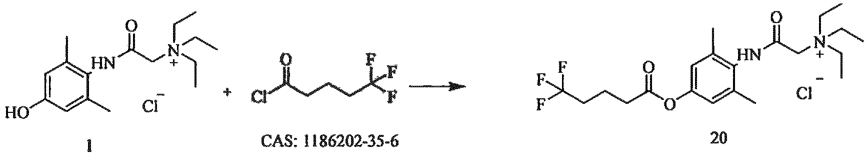

315 mg of compound 1 and 220 mg of 5,5,5-trifluoro-n-valeryl chloride were added to 30 mL of dichloromethane, and 500 mg of pyridine was added dropwise to the mixture under an ice bath. The mixture was removed from the ice bath, and stirred at room temperature for 8 hours. The obtained mixture was evaporated off solvent to dryness under reduced pressure, and the residue was subjected to column chromatography (dichloromethane/methanol=20/1) to obtain 211 mg of a white powder solid, namely compound 20, with a yield of 46.6%.

1HNMR d-DMSO 400Hz: 1.27 (9H, t, J=8Hz), 1.63-1.66(2H, m), 2.16 (6H, s), 2.45-2.51(4H, m), 3.50(6H, q, J= 8Hz), 4.35 (2H, s), 6.83 (2H, s), 10.41 (1H, s).

### Example 21

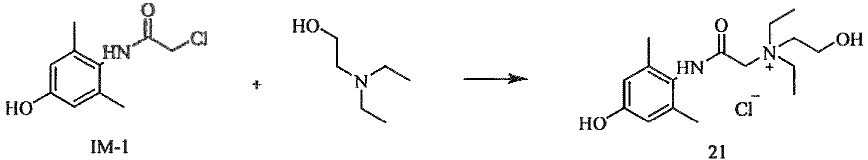

2.13 g of IM-1 and 3 g of 2-diethylaminoethanol were dissolved in 20 mL of acetonitrile, and the obtained mixture was stirred in a sealed tube at 90 °C overnight. The resulting mixture was cooled and evaporated off solvent to dryness under reduced pressure, and the residue was subjected to column chromatography (dichloromethane/methanol=20/1) to obtain 2.09 g of white solid powder, namely compound 21, with a yield of 63.2%.

1HNMR d-DMSO 400Hz: 1.28 (9H,t, J=8Hz), 2.17(6H, s), 3.50-3.52(4H, m), 3.64-3.66(2H, m), 3.98-4.02(2H, m), 4.36 (2H, s), 5.11 (1H, s), 6.81 (2H, s), 9.10 (1H, s), 10.31 (1H, s).

### Example 22

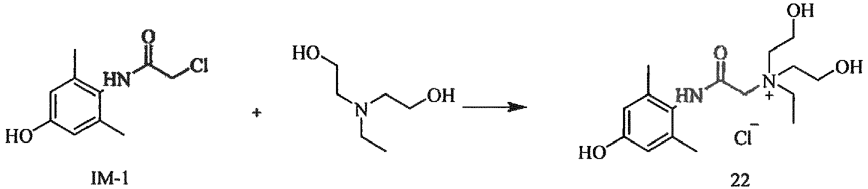

2.13 g of IM-1 and 3 g of dihydroxyethylethylamine were dissolved in 20 mL of acetonitrile, and the obtained mixture was stirred in a sealed tube at 90 °C overnight. The resulting mixture was cooled and evaporated off solvent to dryness under reduced pressure, and the residue was subjected to column chromatography (dichloromethane/methanol=20/1) to obtain 2.18 g of white solid powder, namely compound 22, with a yield of 62.9%.

1HNMR d-DMSO 400Hz: 1.25 (3H, t, J=8Hz), 2.13 (6H, s), 3.43-3.51(6H, m), 3.94-3.99(4H, m), 4.32 (2H, s), 5.67 (2H, s), 6.80 (2H, s), 9.03 (1H, s), 10.11 (1H, s).

### Example 23

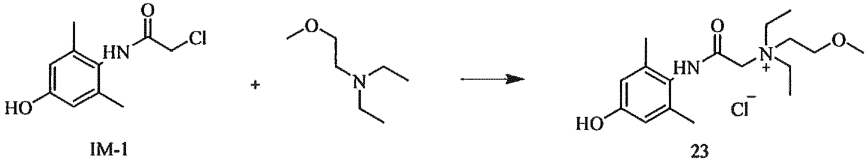

2.13 g of IM-1 and 3 g of methoxyethyldiethylamine were dissolved in 20 mL of acetonitrile, and the obtained mixture was stirred in a sealed tube at 90 °C overnight. The resulting mixture was cooled and evaporated off solvent to dryness under reduced pressure, and the residue was subjected to column chromatography (dichloromethane/methanol=20/1) to obtain 1.68 g of white solid powder, namely compound 23, with a yield of 48.7%.

1HNMR d-DMSO 400Hz: 1.27 (6H, t, J=8Hz), 2.16(6H, s), 3.25 (3H, s), 3.50-3.53(6H, m), 3.82-3.85(2H, m), 4.30 (2H, s), 6.81 (2H, s), 9.01 (1H, s), 10.41 (1H, s).

### Example 24

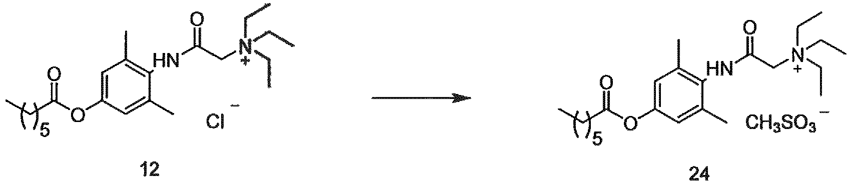

500 mg of compound 12 was dissolved in 100 mL of dichloromethane, and 100 mL of an aqueous solution of 5% sodium methanesulfonate was added to the mixture. The obtained mixture was shaken well for 10 minutes. The organic layer was separated out and evaporated off solvent to dryness under reduced pressure to obtain 315 mg of white solid powder, namely compound 24.

1HNMR d-DMSO 400Hz: 0.88-0.92 (3H, m), 1.26-1.37(15H, m), 1.48-1.64 (2H, m), 2.15(6H, s), 2.52(2H, t, J=8Hz ), 2.78(3H, s), 3.51-3.55(6H, m), 4.41(2H, s), 6.80(2H, s), 10.11(1H, s).

### Example 25

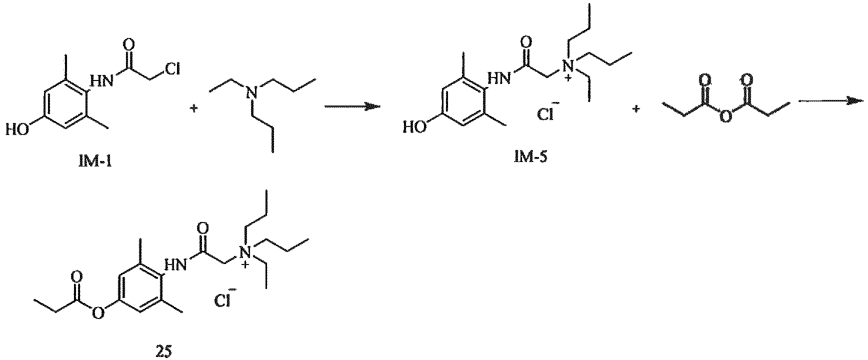

2.13 g of compound IM-1 was dissolved in 30 mL of acetonitrile, 3.6 g of N,N-di-n-propylethylamine was added to the mixture, and the obtained mixture was refluxed and stirred overnight. The resulting mixture was cooled and evaporated off solvent to dryness under reduced pressure, and the residue was subjected to column chromatography (dichloromethane/methanol=20/1) to obtain 1.07 g of white solid powder, namely compound IM-5.

1HNMR d-DMSO 400Hz: 0.92 (6H, t, J=8Hz), 1.23 (3H, t, J=8Hz), 1.68-1.73 (4H, m), 2.16(6H, s), 3.48-3.52(6H , m), 4.40(2H,s), 6.38(2H,s), 9.24(1H,s), 10.82(1H,s).

343 mg of IM-5 was mixed with 1 ml of propionic anhydride, and 3 mL of pyridine was added to the mixture, and the obtained mixture was stirred in a sealed tube at 90 °C overnight. The resulting mixture was cooled and evaporated off solvent to dryness under reduced pressure, and ethyl acetate was added to the residue to precipitate a powder solid. The obtained mixture was filtered to obtain a white solid powder, which was then recrystallized twice with ethyl acetate/ethanol to obtain 168 mg of white solid powder, namely compound 25, with a yield of 42.1%.

1HNMR d-DMSO 400Hz: 0.91 (6H, t, J=8Hz), 1.23-1.27 (6H, m), 1.65-1.72 (4H, m), 2.16(6H, s), 2.50-2.53(2H, m), 3.45-3.51(6H,m), 4.42(2H,s), 6.84(2H,s), 9.01(1H,s), 10.42(1H,s).

### Example 26

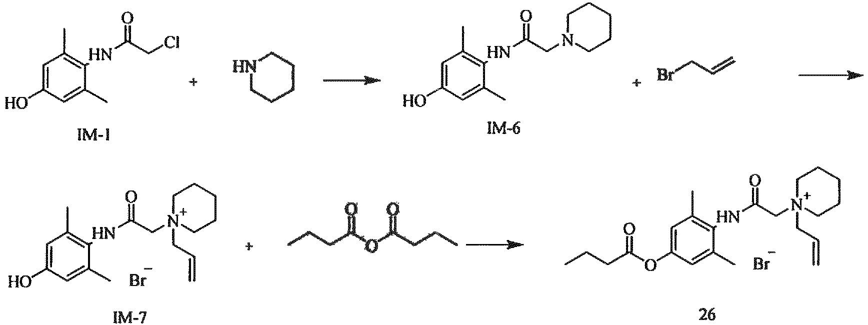

2.13 g of IM-1 was dissolved in 20 mL of acetonitrile, and 0.9 g of piperidine and 2 g of anhydrous potassium carbonate were added to the mixture. The obtained mixture was heated to 50 °C, stirred for 5 hours, and added with allyl bromide to continue to react for 1.5 hours. The reacted mixture was cooled and filtered, the filtrate was evaporated to dryness, and the residue was subjected to column chromatography (dichloromethane/methanol=20/1) to obtain 0.98 g of white solid powder, namely IM-7.

1HNMR d-DMSO 400Hz: 1.58-1.74(6H,m), 2.14(6H,s), 3.16-3.21(4H,m), 3.95(2H,m), 4.40(2H,s), 5.01-5.06(2H ,m), 5.83-5.85 (1H,m), 6.35(2H,s), 9.09 (1H,s), 10.22(1H,s).

383 mg of IM-7 was mixed with 1 mL of n-butyric anhydride, 3 mL of pyridine was added to the mixture, and the obtained mixture was stirred in a sealed tube at 90 °C overnight. The resulting mixture was cooled and evaporated off solvent to dryness under reduced pressure, and ethyl acetate was added to the residue to precipitate a powder solid. The obtained mixture was filtered to obtain a white solid powder, which was then recrystallized twice with ethyl acetate/ethanol to obtain 217 mg of white solid powder, namely compound 26, with a yield of 47.9%.

1HNMR d-DMSO 400Hz: 0.91(3H, t, J=8Hz), 1.54-1.73 (8H, m), 2.13(6H, s), 2.52-2.56 (2H, m), 3.15-3.22(4H, m), 3.96(2H,m), 4.42(2H,s), 5.02-5.08(2H,m), 5.82-5.85(1H,m), 6.83(2H,s), 10.42(1H,s).

### Example 27

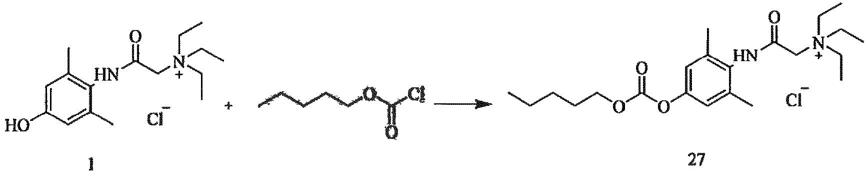

3.14 g of compound 1 was dissolved in 50 mL of acetonitrile, then 1.5 g of anhydrous sodium carbonate was added to the mixture. The obtained mixture was stirred at room temperature for 2 hours, and added with 1.55 g of pentyl chloroformate (CAS: 638-41-5) to react overnight at room temperature. The resulting mixture was filtered, the filtrate was evaporated off solvent to dryness under reduced pressure, and the residue was subjected to column chromatography (dichloromethane/methanol=20/1) to obtain 1.32 g of white powder, namely compound 27, with a yield of 30.8%.

1HNMR d-DMSO 400Hz: 0.88(3H, t, J=8Hz), 1.26-1.35(13H,m), 1.58-1.63(2H,m), 2.14(6H,s), 3.50-3.55(6H,m), 4.32-4.33 (4H, m), 6.88 (2H, s), 10.31 (1H, s).

### Example 28

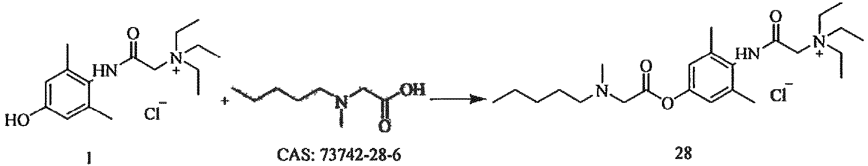

315 mg of compound 1 was dissolved in 50 mL of dichloromethane, 160 mg of N-methyl-N-n-pentyl-aminoacetic acid and 200 mg of 1-ethyl-(3-dimethylaminopropyl) carbodiimide hydrochloride (EDCI) were added to the mixture, and the obtained mixture was stirred at room temperature overnight. The resulting reaction solution was washed twice with water, then the organic layer was separated out, and evaporated off solvent to dryness under reduced pressure, and the residue was subjected to column chromatography to obtain 87 mg of light yellow powder, namely compound 28, with a yield of 11.5%.

1HNMR d-DMSO 400Hz: 0.89 (3H, t, J=8Hz), 1.24-1.33 (15H, m), 2.15 (6H, s), 2.27 (3H, s), 2.39-2.42 (2H, m), 3.51 (6H, q, J=8Hz), 3.55(2H, s), 4.42(2H, s), 6.82 (2H, s), 10.37 (1H, s).

### Example 29

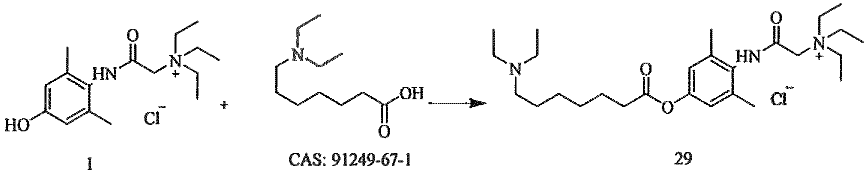

315 mg of compound 1 was dissolved in 50 mL of dichloromethane, 210 mg of 7-diethylaminoheptanoic acid and 200 mg of 1-ethyl-(3-dimethylaminopropyl) carbodiimide hydrochloride (EDCI) were added to the mixture, and the obtained mixture was stirred at room temperature overnight. The resulting reaction solution was washed twice with water, then the organic layer was separated out, and evaporated off solvent to dryness under reduced pressure, and the residue was subjected to column chromatography to obtain 106 mg of light yellow powder, namely compound 29, with a yield of 21.3%.

1HNMR d-DMSO 400Hz: 1.13(6H, t, J=8Hz), 1.22-1.31 (15H, m), 1.62-1.64(2H, m), 2.12 (6H, s), 2.50-2.52(2H, m), 2.99-3.04(6H, m), 3.52(6H, q, J=8Hz), 4.39(2H, s), 6.78 (2H, s), 10.81 (1H, s).

### Example 30

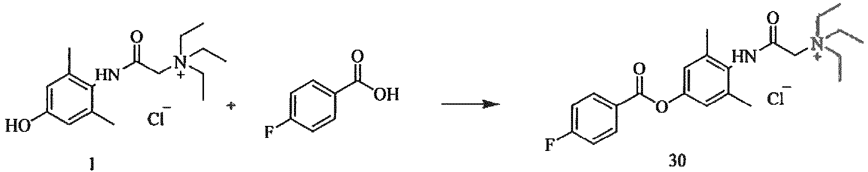

315 mg of compound 1 was dissolved in 50 mL of dichloromethane, 140 mg of 4-fluorobenzoic acid and 200 mg of 1-ethyl-(3-dimethylaminopropyl) carbodiimide hydrochloride (EDCI) were added to the mixture, and the obtained mixture was stirred at room temperature overnight. The resulting reaction solution was washed twice with water, then the organic layer was separated out, and evaporated off solvent to dryness under reduced pressure, and the residue was subjected to column chromatography to obtain 251 mg of white powder, namely compound 30, with a yield of 57.4%.

1HNMR d-DMSO 400Hz: 1.26 (9H, t, J=8Hz), 2.05 (6H, s), 3.50 (6H, q, J=8Hz), 4.32 (2H, s), 6.77 (2H, s), 7.41 -7.45(2H, m), 8.10-8.14(2H, m), 10.57 (1H, s).

### Example 31

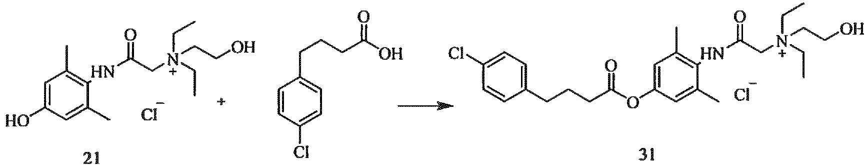

315 mg of compound 1 was dissolved in 50 mL of dichloromethane, 200 mg of 4-p-chlorophenylbutyric acid and 200 mg of 1-ethyl-(3-dimethylaminopropyl) carbodiimide hydrochloride (EDCI) were added to the mixture, and the obtained mixture was stirred at room temperature overnight. The resulting reaction solution was washed twice with water, then the organic layer was separated out, and evaporated off solvent to dryness under reduced pressure, and the residue was subjected to column chromatography to obtain 201 mg of white powder, namely compound 31, with a yield of 39.3%.

1HNMR d-DMSO 400Hz: 1.28 (6H, t, J=8Hz), 1.78-1.80(2H, m), 2.08 (6H, s), 2.50-2.59 (4H, m), 3.48-3.55 (6H, m), 3.94-3.97(2H, m), 4.31(2H, s), 5.67(1H, s), 6.75 (2H, s), 7.23-7.27(2H, m), 7.43-7.46(2H, m), 10.51 (1H, s).

### Example 32

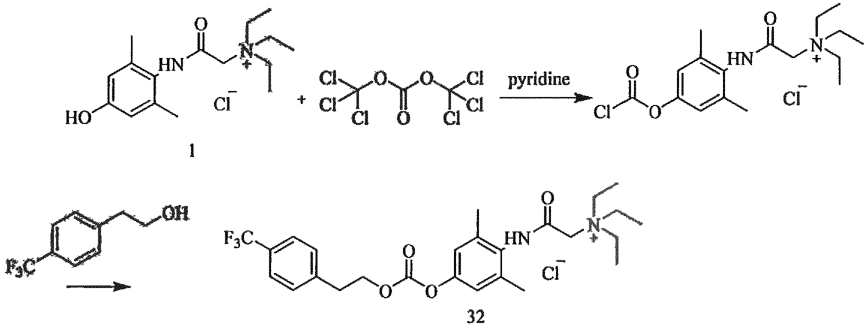

500 mg of compound 1 was dissolved in 30 mL of dichloromethane, and 200 mg of triphosgene was added to the mixture. The resulting mixture was cooled to 0-5°C, and slowly added dropwise with 3 mL of pyridine. The obtained mixture was removed from the ice bath, stirred for 2 hours, then cooled down to 0-5 °C, and added with 300 mg of p-trifluoromethyl phenethyl alcohol. The resulting mixture was stirred for 1 hour, then removed from the ice bath, stirred at room temperature overnight, and added dropwise with dilute hydrochloric acid to adjust the pH to 3. The obtained reaction solution was evaporated off solvent to dryness under reduced pressure, and the residue was subjected to column chromatography (dichloromethane/methanol=10/1) to obtain 179 mg of white powder, namely compound 32, with a yield of 33.7%.

1HNMR d-DMSO 400Hz: 1.27 (9H, t, J=8Hz), 2.08 (6H, s), 3.01-3.05 (2H, m), 3.48 (6H, q, J=8Hz), 4.30 (2H, s),4.51-4.54 (2H, m), 6.89-7.01 (4H, m), 7.49-7.55 (2H, m), 10.31 (1H, s).

### Example 33

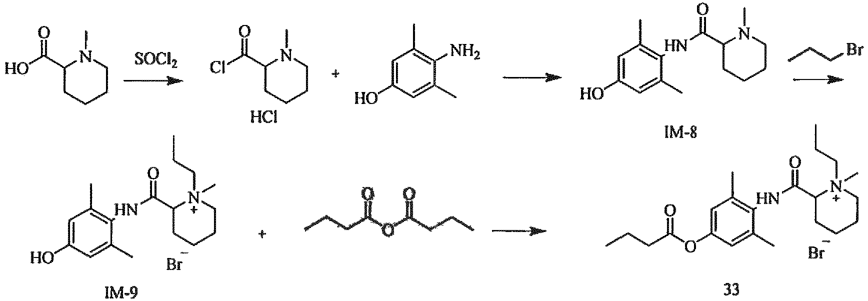

1.43 g of N-methylpiperidine-2-carboxylic acid was mixed with 10 mL of thionyl chloride, and the mixture was heated to 40°C, stirred for 1 hour, and then evaporated off the unreacted thionyl chloride under reduced pressure. The residue was dissolved with 50 mL of methylene chloride to serve as a backup solution. 1.37 g of 4-amino-3,5-dimethylphenol was mixed in 50 mL of dichloromethane, and the mixture was added with 4 g of triethylamine. The obtained reaction solution was cooled in an ice bath, and slowly added dropwise with the backup solution with stirring. After the dropping was completed, the reaction solution was removed from the ice bath, and stirred at room temperature for 2 hours. The reaction solution was then evaporated off solvent to dryness under reduced pressure, and the residue was recrystallized with petroleum ether/ethyl acetate to obtain 0.98 g of a yellow solid, namely IM-8.

1HNMR d-DMSO 400Hz: 1.27-1.33(1H, m), 1.55-1.81(4H, m), 2.09-2.17(8H, m), 2.41(3H,s), 2.65(1H, dd, J=15 Hz), 2.98-3.06(1H, m), 6.47(2H, s), 9.03(1H, s), 10.52(1H, s).

500 mg of IM-8 was dissolved in 10 mL of acetonitrile, 500 mg of anhydrous potassium carbonate and 300 mg of bromopropane were added to the mixture, and the obtained mixture was stirred in a sealed tube at 80°C overnight. The resulting mixture was cooled and filtered, the filtrate was evaporated off solvent to dryness under reduced pressure, and the residue was subjected to column chromatography (dichloromethane/methanol=20/1) to obtain 411 mg of white solid powder, namely IM-9.

1HNMR d-DMSO 400Hz: 0.89 (3H, t, J=8Hz), 1.24-1.32(2H, m), 1.78-1.91(4H, m), 2.07-2.16(8H, m), 3.19-3.31(7H, m), 4.49-4.52(1H, m), 6.45(2H, s), 9.01(1H, s), 10.12(1H, s).

385 mg of IM-9 was mixed with 1 mL of n-butyric anhydride, 3 mL of pyridine was added to the mixture, and the obtained mixture was reacted in a sealed tube at 90°C overnight. The resulting mixture was cooled, and evaporated off solvent to dryness under reduced pressure, and ethyl acetate was added to the residue to precipitate a powder solid. The obtained mixture was filtered to obtain a white solid powder, which was then recrystallized twice with ethyl acetate/ethanol to obtain 209 mg of white solid powder, namely compound 33, with a yield of 45.9%.

1HNMR d-DMSO 400Hz: 0.89-0.92(6H, m), 1.25-1.34(2H, m), 1.78-1.91(6H, m), 2.05-2.14(8H, m), 2.51-2.59(2H, m), 3.16-3.29(7H, m), 4.45-4.48(1H, m), 6.80 (2H, s), 10.72(1H, s).

### Example 34

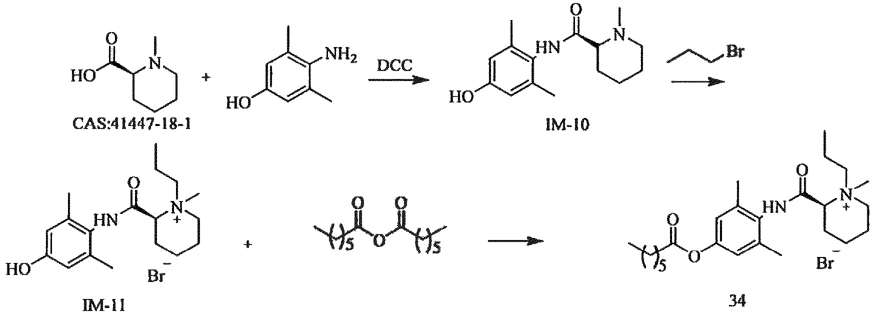

1.43 g of (-)-(2S)-1-methylpiperidine-2-carboxylic acid was dissolved in 50 mL of dichloromethane, then the mixture was added with 2.06 g of dicyclohexylcarbodiimide (DCC) and stirred for half an hour. The obtained mixture was added with 1.37 g of 4-amino-3,5-dimethylphenol and stirred at room temperature for 5 hours. The resulting mixture was filtered, the filtrate was evaporated to dryness under reduced pressure, and the residue was subjected to column chromatography to obtain a yellow solid powder, which was recrystallized with petroleum ether/ethyl acetate to obtain 0.85 g of white solid, namely IM-10.

1HNMR d-DMSO 400Hz: 1.25-1.31(1H, m), 1.56-1.86(4H, m), 2.05-2.12(8H, m), 2.49(3H, s), 2.69-2.93(1H, m), 2.95 -3.03(1H, m), 6.44(2H, s), 9.01(1H, s), 10.12(1H, s).

500 mg of IM-10 was dissolved in 10 mL of acetonitrile, 500 mg of anhydrous potassium carbonate and 300 mg of bromopropane were added to the mixture, and the obtained mixture was stirred in a sealed tube at 80°C overnight. The resulting mixture was cooled and filtered, the filtrate was evaporated off solvent to dryness under reduced pressure, and the residue was subjected to column chromatography (dichloromethane/methanol=20/1) to obtain 367 mg of white solid powder, namely IM-11.

1HNMR d-DMSO 400Hz: 0.92 (3H, t, J=8Hz), 1.22-1.32(2H, m), 1.74-1.90(4H, m), 2.05-2.16(8H, m), 3.14-3.31(7H, m), 4.52(1H, m), 6.41(2H, s), 9.00(1H, s), 10.32(1H, s).

385 mg of IM-11 was mixed with 1 mL of n-heptanoic anhydride, 3 mL of pyridine was added to the mixture, and the obtained mixture was reacted in a sealed tube at 90°C overnight. The resulting mixture was cooled, and evaporated off solvent to dryness under reduced pressure, and ethyl acetate was added to the residue to precipitate a powder solid. The obtained mixture was filtered to obtain a white solid powder, which was then recrystallized twice with ethyl acetate/ethanol to obtain 168 mg of white solid powder, namely compound 34, with a yield of 33.8%.

1HNMR d-DMSO 400Hz: 0.87-0.93(6H, m), 1.24-1.31(8H, m), 1.75-1.92(7H, m), 2.04-2.18(7H, m), 2.53-2.62(2H, m), 3.14-3.28(7H, m), 4.43-4.47(1H, m), 6.83(2H, s), 10.22(1H, s).

### Example 35

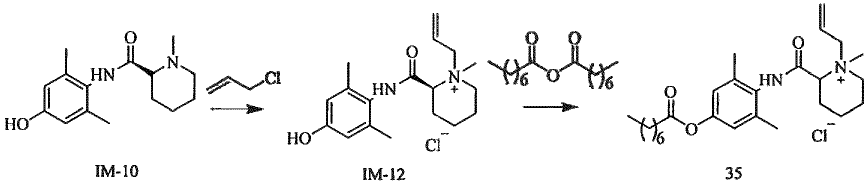

500 mg of IM-10 was dissolved in 10 mL of acetonitrile, 500 mg of anhydrous potassium carbonate and 300 mg of allyl chloride were added to the mixture, and the obtained mixture was stirred in a sealed tube at 65°C overnight. The resulting mixture was cooled and filtered, the filtrate was evaporated off solvent to dryness under reduced pressure, and the residue was subjected to column chromatography (dichloromethane/methanol=20/1) to obtain 269 mg of white solid powder, namely IM-12.

1HNMR d-DMSO 400Hz: 1.21-1.28(2H,m), 1.72-1.76(2H,m), 2.03-2.14(8H,m), 2.93-3.04(1H,m), 3.14-3.31(5H,m), 4.55-4.58(1H, m), 5.02-5.05(2H, m), 5.78-5.82(1H, m), 6.40(2H, s), 9.01(1H, s), 10.12(1H, s).

338 mg of IM-12 was mixed with 1 mL of n-octanoic anhydride, 3 mL of pyridine was added to the mixture, and the obtained mixture was reacted in a sealed tube at 80°C overnight. The resulting mixture was cooled, and evaporated off solvent to dryness under reduced pressure, and ethyl acetate was added to the residue to precipitate a powder solid. The obtained mixture was filtered to obtain a white solid powder, which was then recrystallized twice with ethyl acetate/ethanol to obtain 129 mg of white solid powder, namely compound 35, with a yield of 27.7%.

1HNMR d-DMSO 400Hz: 0.91 (3H, t, J=8Hz), 1.23-1.35(10H, m), 1.67-1.73(4H, m), 1.95-2.14 (8H, m), 2.49-2.51 (2H, m), 3.16-3.41(5H, m), 4.53-4.59 (1H, m), 5.01-5.04(2H, m), 5.75-5.81(1H, m), 6.84 (2H, s), 9.09(1H, s), 10.52(1H, s).

### Example 36

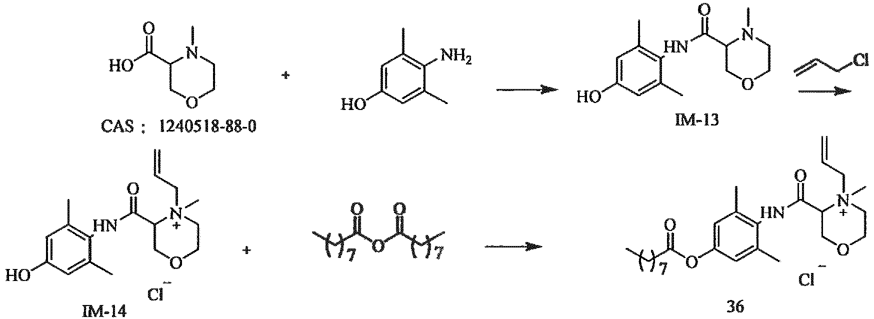

1.45 g of 4-methyl-3-morpholinic acid was dissolved in 50 mL of dichloromethane, and 2.06 g of dicyclohexylcarbodiimide (DCC) was added. The obtained mixture was stirred for half an hour, and 1.37 g of 4-amino-3,5-dimethylphenol was added. The obtained mixture was stirred at room temperature for 5 hours, and filtered. The filtrate was evaporated to dryness under reduced pressure, and the residue was subjected to column chromatography to obtain a white solid powder, which was recrystallized with petroleum ether/ethyl acetate to obtain 0.69 g of white solid, namely IM-13.

1HNMR d-DMSO 400Hz: 2.15(6H, s), 2.43(3H, s), 2.63-2.71(2H, m), 3.67-3.73(3H, m), 3.88-4.04(2H, m), 6.41 (2H), s), 9.05(1H, s), 10.72(1H, s).

500 mg of IM-14 was dissolved in 10 mL of acetonitrile, 500 mg of anhydrous potassium carbonate and 200 mg of allyl chloride were added to the mixture, and the obtained mixture was stirred in a sealed tube at 65°C overnight. The resulting mixture was cooled and filtered, the filtrate was evaporated off solvent to dryness under reduced pressure, and the residue was subjected to column chromatography (dichloromethane/methanol=20/1) to obtain 391 mg of white solid powder, namely IM-14.

1HNMR d-DMSO 400Hz: 2.13 (6H, s), 3.34-3.51(5H, m), 3.77-4.01(5H, m), 4.25-4.28(1H, m), 4.87-5.11(3H, m), 5.74 -5.79(1H, m), 6.38(2H, s), 9.11(1H, s), 10.32(1H, s).

340 mg of IM-14 was mixed with 1 mL of n-nonanoic anhydride, 3 mL of pyridine was added to the mixture, and the obtained mixture was reacted in a sealed tube at 90°C overnight. The resulting mixture was cooled, and evaporated off solvent to dryness under reduced pressure, and ethyl acetate was added to the residue to precipitate a powder solid. The obtained mixture was filtered to obtain a white solid powder, which was then recrystallized twice with ethyl acetate/ethanol to obtain 98 mg of white solid powder, namely compound 36, with a yield of 20.4%.

1HNMR d-DMSO 400Hz: 0.87-0.91 (3H, t, J=8Hz), 1.23-1.35(10H, m), 1.63-1.66(2H, m), 2.17(6H, s), 2.53-2.58(2H, m), 3.34-3.58(5H, m), 3.75-4.00(5H, m), 4.23-4.28(1H, m), , 4.85-5.09(3H, m), 5.72-5.75(1H, m), 6.85 (2H, s), 10.71(1H, s).

### Example 37

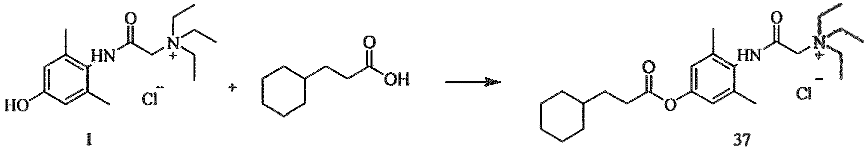

315 mg of compound 1 was dissolved in 30 mL of dichloromethane, and 156 mg of cyclohexylpropionic acid was added to the mixture. The mixture was stirred. 210 mg of DCC was dissolved in 10 mL of dichloromethane, and the obtained mixture was added dropwise to the above reaction solution with stirring. After the dropping was completed, the resulting reaction solution was reacted at room temperature overnight. The reacted mixture was filtered, the filtrate was evaporated off solvent to dryness under reduced pressure, and the residue was subjected to column chromatography (dichloromethane/methanol=20/1) to obtain 266 mg of white solid powder, namely compound 37, with a yield of 60.6%.

1HNMR d-DMSO 400Hz: 1.23 (9H, t, J=8 Hz), 1.53-1.66(13H, m), 2.14(6H, s), 2.53-2.56(2H, m), 3.58 (6H, q, J =8Hz), 4.36(2H, s), 6.75(2H, s), 10.31(1H, s).

### Example 38

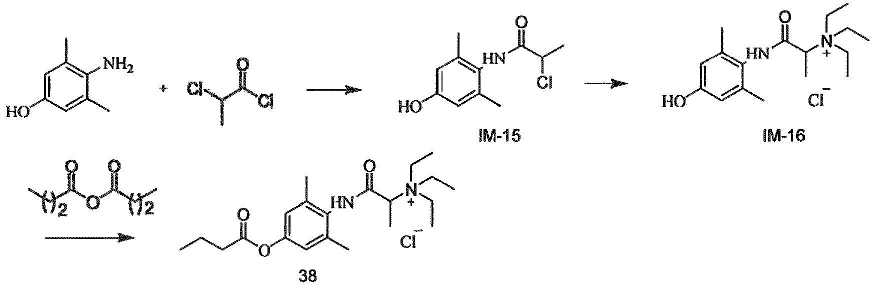

13.7 g of 4-amino-3,5-dimethylphenol and 40 g of triethylamine were dissolved in 150 mL of dichloromethane, and 12.7 g of 2-chloropropionyl chloride was slowly added dropwise to the mixture under an ice bath. After the dropping was completed, the reaction solution was stirred to react for 2 hours, and then the reacted solution was evaporated off solvent to dryness under reduced pressure. The residue was added with 150 mL of ethyl acetate. The organic layer was washed with 50 mL of dilute hydrochloric acid, separated out, and evaporated off ethyl acetate to dryness under reduced pressure to obtain brown-black oil, which was then purified by column chromatography (petroleum ether/ethyl acetate=3/1) to obtain 4.11 g of a yellow solid powder (IM-15), with a yield of 18%.

600 mg of IM-15 was dissolved in 5 mL of absolute ethanol, and 2 mL of triethylamine was added to the mixture. The obtained mixture was reacted in a sealed tube at an external temperature of 95 °C for 8 hours to precipitate a white solid. The obtained mixture was cooled, then added with 5 mL of ethanol, and added with 2 drops of concentrated hydrochloric acid to adjust the pH to below 3, and then the mixture was evaporated off ethanol to dryness under reduced pressure to obtain a white solid powder, which was then purified by column chromatography (dichloromethane/methanol=10/1) to obtain 311 mg of white powder, namely IM-16, with a yield of 35.9%.

1HNMR d-DMSO 400Hz: 1.26 (9H, t, J=8Hz), 1.53 (3H, d, J=Hz), 2.09 (6H, s), 3.50 (6H, q, J=8Hz), 4.58-4.61 ( 1H, m), 6.45 (2H, s), 9.02 (1H, s), 10.11 (1H, s).

329 mg of IM-16 was mixed with 1 mL of n-butyric anhydride, and 3 mL of pyridine was added to the mixture. The obtained mixture was reacted in a sealed tube at 90 °C overnight. The resulting mixture was cooled and evaporated off solvent to dryness under reduced pressure, and ethyl acetate was added to the residue to precipitate a powder solid. The obtained mixture was filtered to obtain a white solid powder, which was then recrystallized twice with ethyl acetate/ethanol to obtain 105 mg of white solid powder, namely compound 38, with a yield of 26.3%.

1HNMR d-DMSO 400Hz: 1.02(3H, t, J=8Hz), 1.24 (9H, t, J=8Hz), 1.50(3H, d, J=Hz), 1.65-1.69(2H, m), 2.04 ( 6H, s), 2.52-2.58(2H, m), 3.51 (6H, q, J=8Hz), 4.55-4.61 (1H, m), 6.87 (2H, s), 10.61 (1H, s).

### Example 39

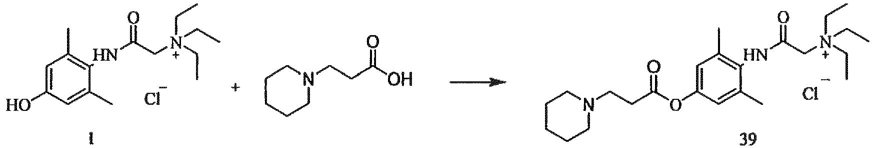

315 mg of compound 1 was dissolved in 30 mL of dichloromethane, and 157 mg of 1-piperidine propionic acid was added to the mixture. The mixture was stirred. 210 mg of DCC was dissolved in 10 mL of dichloromethane, and the obtained mixture was added dropwise to the above reaction solution with stirring. After the dropping was completed, the resulting reaction solution was reacted at room temperature overnight. The reacted mixture was filtered, the filtrate was evaporated off solvent to dryness under reduced pressure, and the residue was subjected to column chromatography (dichloromethane/methanol=20/1) to obtain 241 mg of white solid powder, namely compound 39, with a yield of 53.1%.

1HNMR d-DMSO 400Hz: 1.25 (9H, t, J=8 Hz), 1.41-1.51(6H, m), 2.12(6H, s), 2.43-2.47(4H, m), 2.61-2.65 (2H, m)), 3.51 (6H, q, J=8Hz), 3.75-3.79(2H, m), 4.34 (2H, s), 6.81 (2H, s), 10.11(1H, s).

### Example 40

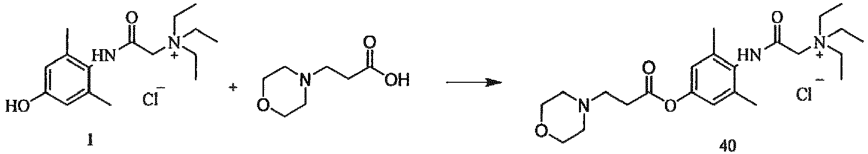

315 mg of compound 1 was dissolved in 30 mL of dichloromethane, and 159 mg of 3-(4-morpholinyl)propionic acid was added to the mixture. The mixture was stirred. 210 mg of DCC was dissolved in 10 mL of dichloromethane, and the obtained mixture was added dropwise to the above reaction solution with stirring. After the dropping was completed, the resulting reaction solution was reacted at room temperature overnight. The reacted mixture was filtered, the filtrate was evaporated off solvent to dryness under reduced pressure, and the residue was subjected to column chromatography (dichloromethane/methanol=20/1) to obtain 257 mg of white solid powder, namely compound 40, with a yield of 56.4%.

1HNMR d-DMSO 400Hz: 1.26 (9H, t, J=8 Hz), 2.14 (6H, s), 2.23-2.28 (4H, m), 2.60-2.67 (2H, m), 3.53 (6H, q, J =8Hz), 3.55-3.61(4H, m), 3.74-3.77(2H, m), 4.36 (2H, s), 6.83 (2H, s), 10.62(1H, s).

### Example 41

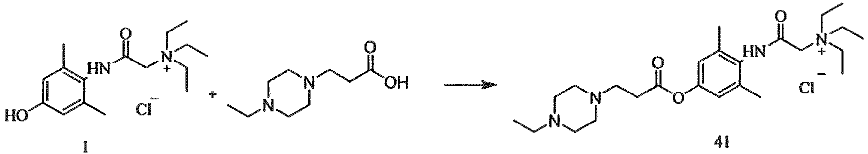

315 mg of compound 1 was dissolved in 30 mL of dichloromethane, and 186 mg of 3-(4-ethylpiperazin-1-yl)-propionic acid (CAS: 799262-18-3) was added to the mixture. The mixture was stirred. 210 mg of DCC was dissolved in 10 mL of dichloromethane, and the obtained mixture was added dropwise to the above reaction solution with stirring. After the dropping was completed, the resulting reaction solution was reacted at room temperature overnight. The reacted mixture was filtered, the filtrate was evaporated off solvent to dryness under reduced pressure, and the residue was subjected to column chromatography (dichloromethane/methanol=20/1) to obtain 187 mg of light yellow solid powder, namely compound 41, with a yield of 38.7%.

1HNMR d-DMSO 400Hz: 1.01(3H, t, J=8Hz), 1.24 (9H, t, J=8 Hz), 2.13(6H, s), 2.29-2.38(10H, m), 2.61-2.66 (2H), m), 3.55 (6H, q, J=8Hz), 3.73-3.78(2H, m), 4.35 (2H, s), 6.80 (2H, s), 10.18(1H, s).

### Example 42

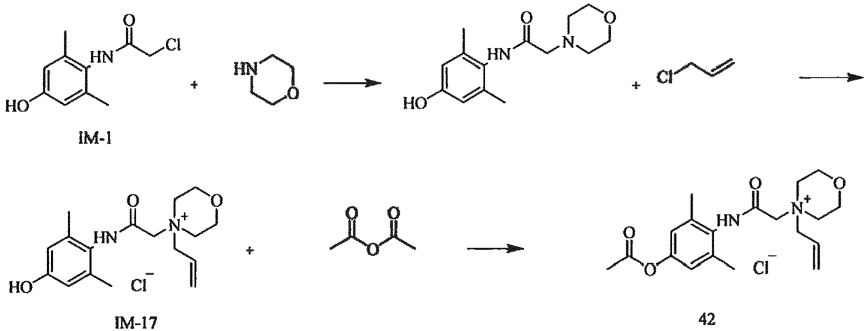

2.13 g of IM-1 was dissolved in 20 mL of acetonitrile, and the mixture was added with 0.88 g of morpholine and 2 g of anhydrous potassium carbonate. The obtained mixture was heated to 50 °C, stirred for 5 hours, and then added with propenyl chloride to continue to react for 6 hours. The reacted mixture was cooled and filtered, the filtrate was evaporated to dryness under reduced pressure, and the residue was subjected to column chromatography (dichloromethane/methanol=20/1) to obtain 1.05 g of white solid powder, namely IM-17.

1HNMR d-DMSO 400Hz: 2.16(6H, s), 3.39-3.45(4H, m), 3.96-4.03 (6H, m), 4.32(2H, s), 5.03-5.07(2H, m), 5.72-5.77 (1H, m), 6.35(2H, s), 9.09(1H, s), 10.22(1H, s).

340 mg of IM-17 was mixed with 1 mL of acetic anhydride, and 3 mL of pyridine was added to the mixture. The obtained mixture was stirred in a sealed tube at 90°C overnight. The resulting mixture was cooled, and evaporated off solvent to dryness under reduced pressure, and ethyl acetate was added to the residue to precipitate a powder solid. The obtained mixture was filtered to obtain a white solid powder, which was then recrystallized twice with ethyl acetate/ethanol to obtain 208 mg of white solid powder, namely compound 42, with a yield of 54.3%.

1HNMR d-DMSO 400Hz: 2.14(6H, s), 2.37(3H, s), 3.37-3.44(4H, m), 3.95-4.01 (6H, m), 4.35(2H, s), 5.04-5.09(2H, m), 5.71-5.77 (1H, m), 6.85(2H, s), 9.01 (1H, s), 10.82(1H, s).

### Example 43

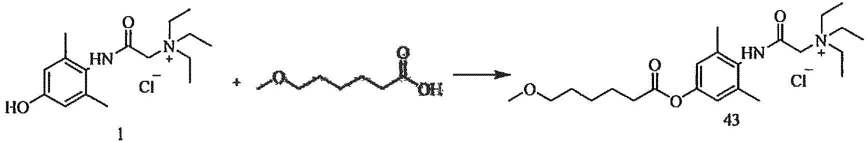

315 mg of compound 1 was dissolved in 20 mL of dichloromethane, and 146 mg of 6-methoxyhexanoic acid was added to the mixture. The mixture was stirred. 210 mg of DCC was dissolved in 10 mL of dichloromethane, and the obtained mixture was added dropwise to the above reaction solution with stirring. After the dropping was completed, the resulting reaction solution was reacted at room temperature overnight. The reacted mixture was filtered, the filtrate was evaporated off solvent to dryness under reduced pressure, and the residue was subjected to column chromatography (dichloromethane/methanol=20/1) to obtain 155 mg of white solid powder, namely compound 43, with a yield of 35.0%.

1HNMR d-DMSO 400Hz: 1.24-1.29(11H, m), 1.54-1.57(2H, m), 1.64-1.68(2H, m), 2.14(6H, s), 2.51-2.55(2H, m), 3.21 -3.38(11H, m), 4.33(2H, s), 6.83(2H, s), 10.17(1H, s).

### Example 44

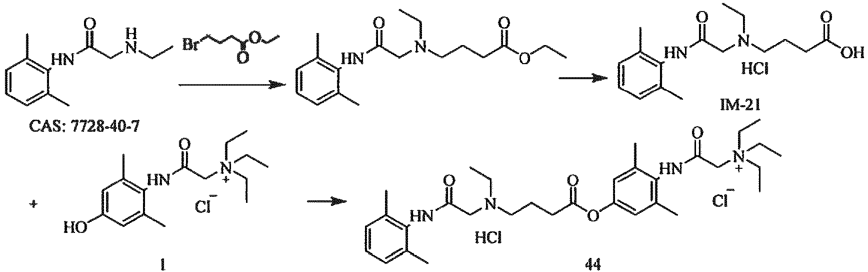

2.06 g of N-(2,6-dimethylaniline)-2-ethylaminoacetamide was dissolved in 100 mL of acetonitrile, and 1.95 g of ethyl 4-bromobutyrate and 2 g of anhydrous potassium carbonate were added to the mixture. The obtained mixture was stirred at 40 °C for 2 hours, cooled and filtered. The filtrate was evaporated to dryness under reduced pressure, and the residue was added with 50 mL of methanol and added dropwise with 5 mL of an aqueous solution of 10% sodium hydroxide. The resulting mixture was stirred at room temperature for 2 hours. 6N hydrochloric acid was used to adjust the pH of the mixture to 3. The obtained mixture was evaporated off solvent under reduced pressure, and the residue was subjected to column chromatography (dichloromethane/methanol=20/1) to obtain 1.27 g of white solid, namely IM-21, with a yield of 38.6%.

1HNMR d-DMSO 400Hz: 1.54(3H, t, J=8Hz), 1.97-2.32(10H, m), 3.38-3.95(4H, m), 4.28(2H, s), 7.09-7.18(3H, m), 10.12(1H, s), 10.49(1H, s), 12.9(1H, s, broad).

329 mg of IM-21 was dissolved in 20 mL of dichloromethane, 315 mg of compound 1 was added to the mixture. The mixture was stirred. 210 mg of DCC was dissolved in 10 mL of dichloromethane, and the obtained mixture was added dropwise to the above reaction solution with stirring. After the dropping was completed, the resulting reaction solution was reacted at room temperature overnight. The reacted mixture was filtered, the filtrate was evaporated off solvent to dryness under reduced pressure, and the residue was subjected to column chromatography (dichloromethane/methanol=20/1) to obtain 103 mg of white solid powder, namely compound 44, with a yield of 16.5%.

1HNMR d-DMSO 400Hz: 1.23(9H, t, J=8Hz), 1.56(3H, t, J=8Hz), 2.09-2.18(14H, m), 2.53-2.58(2H, m), 3.41-3.51( 10H,m ), 4.28(2H, s), 4.31(2H, m), 6.82(2H, s), 7.12-7.19(3H, m), 10.12(1H, s), 10.67(1H, s).

### Example 45

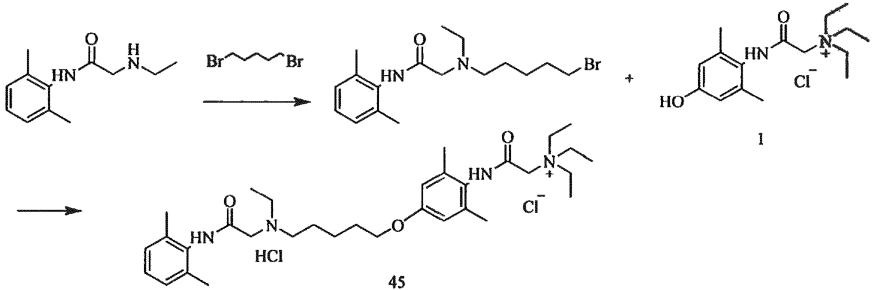

2.06 g of N-(2,6-dimethylaniline)-2-ethylaminoacetamide was dissolved in 100 mL of acetonitrile, and 2.3 g of 1,5-dibromopentane and 3 g of anhydrous potassium carbonate were added to the mixture. The obtained mixture was stirred at 40 °C for 2 hours, and added with 3.15 g of compound 1. The resulting mixture was continued to be stirred overnight, then cooled and filtered. The filtrate was evaporated to dryness under reduced pressure, and the residue was added with 50 mL of methanol, and added dropwise with 5 mL of an aqueous solution of 10% sodium hydroxide. The resulting mixture was stirred at room temperature for 2 hours. 6N hydrochloric acid was used to adjust the pH of the mixture to 3. The obtained mixture was evaporated off solvent under reduced pressure, and the residue was subjected to column chromatography (dichloromethane/methanol=20/1) to obtain 0.93 g of white solid, namely compound 45, with a yield of 14.8%.

1HNMR d-DMSO 400Hz: 1.23-1.28(11H,m), 1.59-1.81(7H,m), 2.17(6H,s), 3.31-3.41(10H,m), 3.99-4.07(2H,m), 4.24 (2H, s), 6.81(2H, s), 7.16-7.18(3H, m), 10.02(1H, s), 10.45(1H, s).

### Example 46

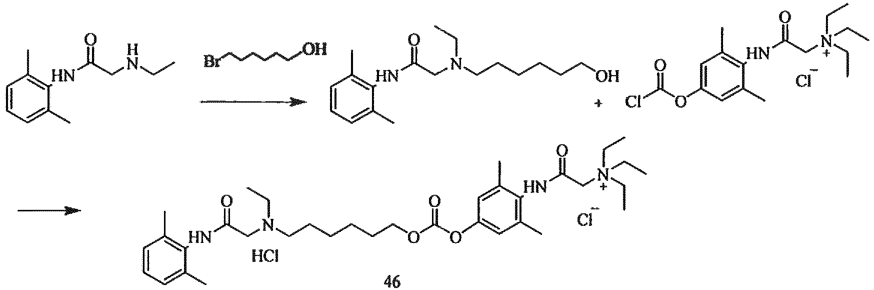

2.06 g of N-(2,6-dimethylaniline)-2-ethylaminoacetamide was dissolved in 100 mL of acetonitrile, and 1.87 g of 6-bromo-1-hexanol and 3 g of anhydrous potassium carbonate were added to the mixture. The obtained mixture was stirred at 70 °C for 5 hours, cooled and filtered, and the filtrate was evaporated to dryness to obtain a crude product for later use.

3.77 g of compound 1 was dissolved in 100 mL of dichloromethane, and 1.4 g of triphosgene was added to the mixture. The obtained mixture was cooled to 0-5 °C, and slowly added dropwise with 30 mL of pyridine. The resulting mixture was removed from the ice bath, stirred for 2 hours, then cooled down to 0-5 °C, and added with the above-mentioned crude product. The obtained mixture was stirred for 1h, removed from the ice bath, stirred at room temperature overnight, and then added dropwise with dilute hydrochloric acid to adjust the pH to 3. The obtained mixture was evaporated off solvent to dryness under reduced pressure, and the crude product was subjected to column chromatography (dichloromethane/methanol=20/1) to obtain 1.12 g of white solid, namely compound 46, with a yield of 16.4%.

1HNMR d-DMSO 400Hz: 1.24-1.31(11H,m), 1.41-1.46(2H,m), 1.59-1.62(3H,m), 1.69-1.73(4H,m), 2.14(6H,s), 2.17 (6H,s), 3.29-3.40(10H, m), 4.21-4.28(4H, m), 6.80(2H, s), 7.13-7.18(3H, m), 10.31(1H, s), 10.45(1H , s).

### Example 47

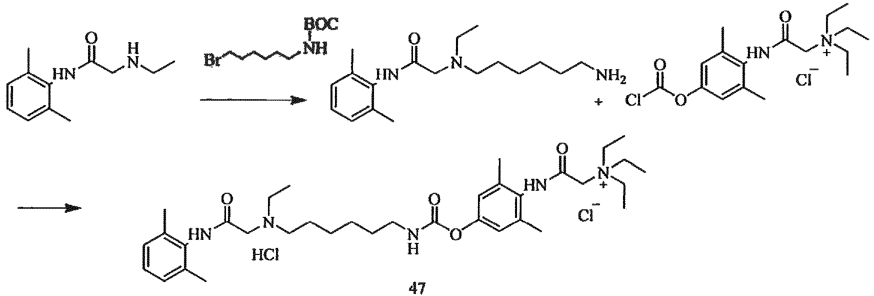

2.06 g of N-(2,6-dimethylaniline)-2-ethylaminoacetamide was dissolved in 100 mL of acetonitrile, and 2.80 g of N-BOC-6-bromohexylamine (142356-33-0) and 3 g of anhydrous potassium carbonate were added to the mixture. The obtained mixture was stirred at 70 °C for 5 hours, cooled and filtered. The filtrate was evaporated to dryness, and the residue was dissolved in 50 mL of ethyl acetate. The resulting mixture was introduced with dry hydrogen chloride gas for 10 minutes, stirred at room temperature for 2 hours, and evaporated off solvent to dryness under reduced pressure to obtain a crude product for later use.

3.77 g of compound 1 was dissolved in 100 mL of dichloromethane, and 1.4 g of triphosgene was added to the mixture. The obtained mixture was cooled to 0-5 °C, and slowly added dropwise with 30 mL of pyridine. The resulting mixture was removed from the ice bath, stirred for 2 hours, then cooled down to 0-5 °C, and added with the above-mentioned crude product. The obtained mixture was stirred for 1h, removed from the ice bath, stirred at room temperature overnight, and then dilute hydrochloric acid was added dropwise to adjust the pH of the mixture to 3. The obtained mixture was evaporated off solvent to dryness under reduced pressure, and the crude product was subjected to column chromatography (dichloromethane/methanol=20/1) to obtain 1.98 g of white solid, namely compound 47, with a yield of 29.0%.

1HNMR d-DMSO 400Hz: 1.23-1.33(13H,m), 1.45-1.53(5H,m), 1.69-1.72(2H,m), 2.16(6H,s), 2.19(6H,s), 3.27-3.39 (12H, m), 4.19-4.25(4H, m), 6.58(1H, s), 6.80(2H, s), 7.11-7.15(3H, m), 10.28(1H, s), 10.44(1H, s).

### Example 48

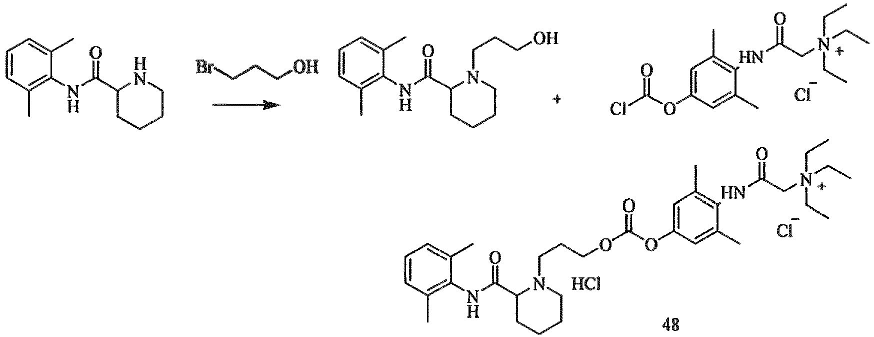

2.32 g of N-(2,6-dimethylphenyl)piperidine-2-carboxamide (CAS: 15883-20-2) was dissolved in 100 mL of acetonitrile, and 1.39 g of 3-bromo-1-propanol and 3 g of anhydrous potassium carbonate were added to the mixture. The obtained mixture was stirred at 70°C for 5 hours, cooled and filtered, and the filtrate was evaporated to dryness to obtain a crude product for later use.

3.77 g of compound 1 was dissolved in 100 mL of dichloromethane, and 1.4 g of triphosgene was added to the mixture. The obtained mixture was cooled to 0-5 °C, and slowly added dropwise with 30 mL of pyridine. The resulting mixture was removed from the ice bath, stirred for 2 hours, then cooled down to 0-5 °C, and added with the above-mentioned crude product. The obtained mixture was stirred for 1h, removed from the ice bath, stirred at room temperature overnight, and then dilute hydrochloric acid was added dropwise to adjust the pH of the mixture to 3. The obtained mixture was evaporated off solvent to dryness under reduced pressure, and the crude product was subjected to column chromatography (dichloromethane/methanol=20/1) to obtain 1.31 g of white solid, namely compound 48, with a yield of 19.61 %.

1HNMR d-DMSO 400Hz: 1.27-1.54(11H, m), 1.69-1.73(2H, m), 1.93-2.21(16, m), 3.25-3.37(10H, m), 4.23-4.29(4H, m), 4.55-4.59(1H, m), 6.81(2H, s), 7.08-7.15(3H, m), 10.12(1H, s), 10.47(1H, s).

### Example 49

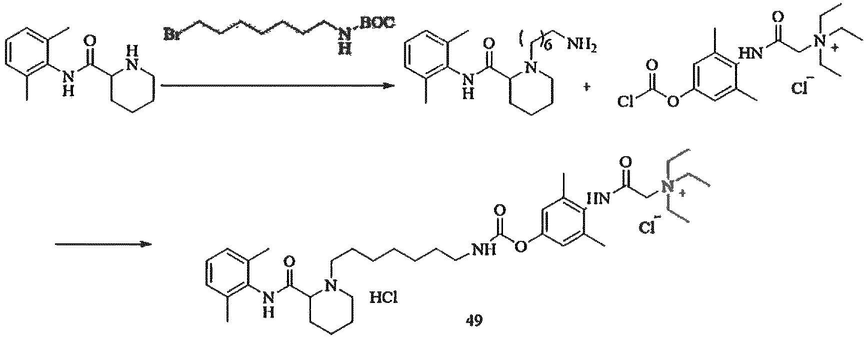

2.32 g of N-(2,6-dimethylphenyl)piperidine-2-carboxamide (CAS: 15883-20-2) was dissolved in 100 mL of acetonitrile, and 2.94 g of N-BOC-7-bromoheptylamine (142356-34-1) and 3 g of anhydrous potassium carbonate were added to the mixture. The obtained mixture was stirred at 70°C for 5 hours, cooled and filtered. The filtrate was evaporated to dryness, and the residue was dissolved in 50 mL of ethyl acetate. The resulting mixture was introduced with dry hydrogen chloride gas for 10 minutes, and stirred at room temperature for 2 hours, and the obtained mixture was evaporated off solvent to dryness under reduced pressure to obtain a crude product for later use.

3.77 g of compound 1 was dissolved in 100 mL of dichloromethane, and 1.4 g of triphosgene was added to the mixture. The obtained mixture was cooled to 0-5 °C, and slowly added dropwise with 30 mL of pyridine. The resulting mixture was removed from the ice bath, stirred for 2 hours, then cooled down to 0-5 °C, and added with the above-mentioned crude product. The obtained mixture was stirred for 1h, removed from the ice bath, stirred at room temperature overnight, and then dilute hydrochloric acid was added dropwise to adjust the pH of the mixture to 3. The obtained mixture was evaporated off solvent to dryness under reduced pressure, and the crude product was subjected to column chromatography (dichloromethane/methanol=20/1) to obtain 2.11 g of white solid, namely compound 49, with a yield of 29.2%.

1HNMR d-DMSO 400Hz: 1.27-1.37(17H, m), 1.59-1.74(4H, m), 1.91-2.23(14, m), 3.25-3.39(12H, m), 4.29(2H, s), 4.52 -4.56(1H, m), 6.80(2H, s), 7.01(1H,s), 7.08-7.15(3H, m), 10.01(1H, s), 10.36(1H,s).

### Example 50

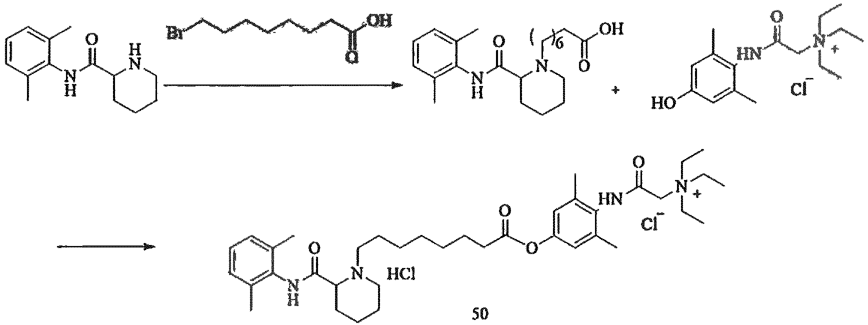

2.32 g of N-(2,6-dimethylphenyl)piperidine-2-carboxamide (CAS: 15883-20-2) was dissolved in 100 mL of acetonitrile, and 2.23 g of 8-bromo-octanoic acid (17696-11 -6) and 3 g of anhydrous potassium carbonate were added to the mixture. The obtained mixture was stirred at 70°C for 5 hours, cooled and filtered, and the filtrate was added with dilute hydrochloric acid to adjust the pH to 3. The resulting mixture was evaporated off solvent to dryness to obtain a crude product for later use.

3.77 g of compound 1 was dissolved in 100 mL of dichloromethane, and the above-mentioned crude product and 2.1 g of dicyclohexylcarbodiimide (DCC) were added to the mixture. The mixture was stirred overnight. The obtained mixture was filtered the next day, the filtrate was evaporated off solvent to dryness under reduced pressure, and the obtained crude product was subjected to column chromatography (dichloromethane/methanol=20/1) to obtain 1.58 g of white solid, namely compound 50, with a yield of 22.3%.

1HNMR d-DMSO 400Hz: 1.25-1.38(17H, m), 1.61-1.75(6H, m), 1.88-2.17(14, m), 2.43-2.49(2H, m), 3.21-3.33(10H, m), 4.21(2H, s), 4.51-4.55(1H, m), 6.82(2H, s), 7.05-7.13(3H, m), 10.09(1H, s), 10.32(IH, s).

### Example 51

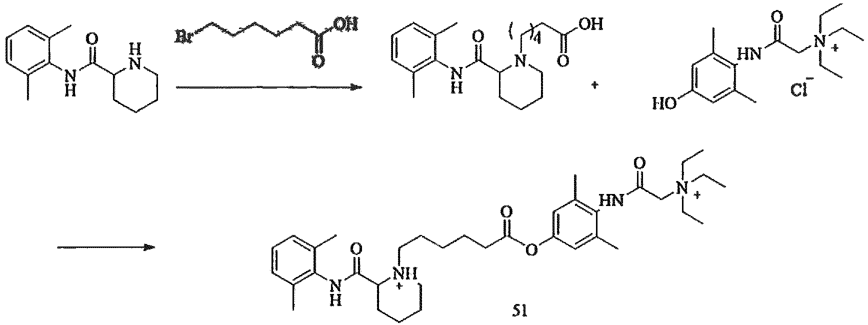

2.32 g of N-(2,6-dimethylphenyl)piperidine-2-carboxamide (CAS: 15883-20-2) was dissolved in 100 mL of acetonitrile, and 2.23 g of 6-bromo-hexanoic acid (4224- 70-8) and 3 g of anhydrous potassium carbonate were added to the mixture. The obtained mixture was stirred at 70°C for 5 hours, cooled and filtered, and then the filtrate was added with dilute hydrochloric acid to adjust the pH to 3, and the resulting mixture was evaporated off solvent to dryness to obtain a crude product for later use.

3.77 g of compound 1 was dissolved in 100 mL of dichloromethane, and the above-mentioned crude product and 2.1 g of dicyclohexylcarbodiimide (DCC) were added to the mixture. The mixture was stirred overnight. The obtained mixture was filtered the next day, the filtrate was evaporated off solvent to dryness under reduced pressure, and the obtained crude product was subjected to column chromatography (dichloromethane/methanol=20/1) to obtain 1.66 g of white solid, namely compound 51, with a yield of 24.4%.

1HNMR d-DMSO 400Hz: 1.22-1.39(13H, m), 1.65-1.76(6H, m), 1.89-2.21(14, m), 2.49-2.54(2H, m), 3.25-3.36(10H, m), 4.23(2H, s), 4.50-4.57(1H, m), 6.81(2H, s), 7.07-7.14(3H, m), 10.19(1H, s), 10.42(1H, s).

### Example 52

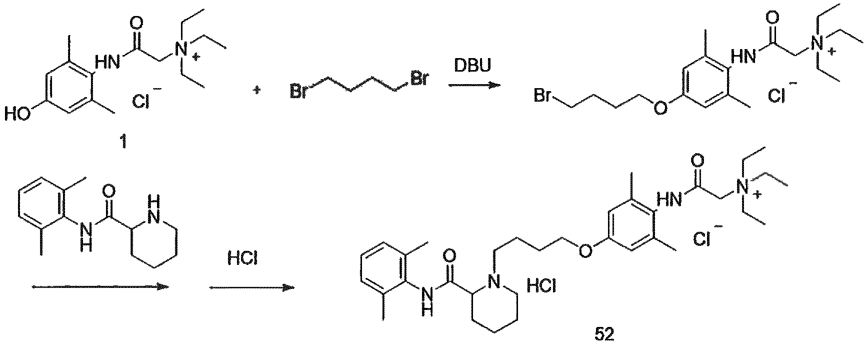

3.15 g of compound 1 was dissolved in 20 mL of DMF, and 3 g of DBU (CAS: 6674-22-2) and 2.16 g of 1,4-dibromobutane were added to the mixture. The obtained mixture was stirred at 70 °C for 6 hours, and added with 1.23 g of 2-pipecolinic acid (CAS: 4043-87-2), and the mixture was stirred to continue to react for 6 hours. 6N hydrochloric acid was added to adjust the pH of the reaction solution to 3, The obtained mixture was evaporated off solvent to dryness under reduced pressure, and the residue was subjected to column chromatography (dichloromethane/methanol=20/1) to obtain 0.78 g of white solid, namely compound 52, with a yield of 12.2%.

1HNMR d-DMSO 400Hz: 1.24-1.33(11H, m), 1.69-1.74(6H, m), 1.92-2.23(14, m), 3.21-3.32(10H, m), 4.01-4.07(2H, m) ,4.21(2H, s), 4.52-4.56(1H, m), 6.80(2H, s), 7.08-7.19(3H, m), 10.21(1H, s), 10.82(1H, s).

### Example 53

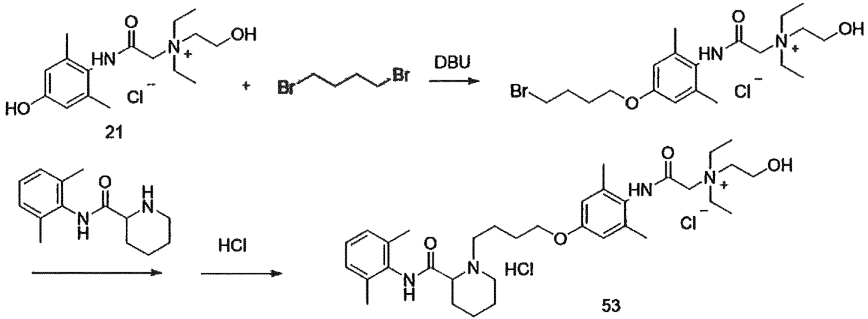

3.30 g of compound 21 was dissolved in 20 mL of DMF, and 3 g of DBU (CAS: 6674-22-2) and 2.16 g of 1,4-dibromobutane were added to the mixture. The obtained mixture was stirred at 70 °C for 6 hours, and added with 1.23 g of 2-pipecolinic acid (CAS: 4043-87-2), and the resulting mixture was stirred to continue to react for 6 hours. 6N hydrochloric acid was added to adjust the pH of the reaction solution to 3. The obtained mixture was evaporated off solvent to dryness under reduced pressure, and the residue was subjected to column chromatography (dichloromethane/methanol=20/1) to obtain 0.93 g of white solid, namely compound 53, with a yield of 14.2%.

1HNMR d-DMSO 400Hz: 1.21-1.31(8H, m), 1.67-1.73(6H, m), 1.90-2.24(14, m), 3.20-3.42(10H, m), 3.95-4.24(7H, m), 4.49-4.53(1H, m), 6.76(2H, s), 7.05-7.15(3H, m), 10.11(1H, s), 10.52(1H, s).

### Example 54

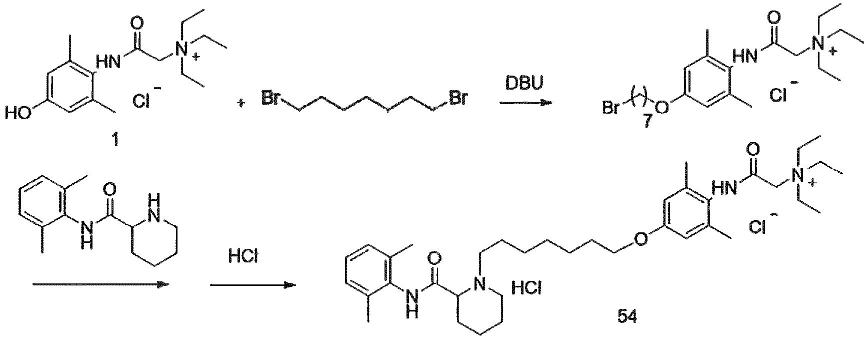

3.15 g of compound 1 was dissolved in 20 mL of DMF, and 3 g of DBU (CAS: 6674-22-2) and 2.58 g of 1,7-dibromoheptane were added to the mixture. The obtained mixture was stirred at 80 °C for 6 hours, and added with 1.23 g of 2-pipecolinic acid (CAS: 4043-87-2), and the mixture was stirred to continue to react for 6 hours. 6N hydrochloric acid was added to adjust the pH of the reaction solution to 3. The obtained mixture was evaporated off solvent to dryness under reduced pressure, and the residue was subjected to column chromatography (dichloromethane/methanol=20/1) to obtain 0.93 g of white solid, namely compound 54, with a yield of 13.7%.

1HNMR d-DMSO 400Hz: 1.20-1.24(10H, m), 1.25-1.29(5H, m), 1.41-1.44(2H, m), 1.70-1.74(6, m), 1.91-2.14(14H, m), 3.15-3.29(10H, m), 4.01-4.09(2H, m), 4.19 (2H, s), 4.52-4.57(1H, m), 6.75(2H, s), 7.05-7.11(3H, m), 10.21(1H,s), 10.62(1H,s).

### Example 55

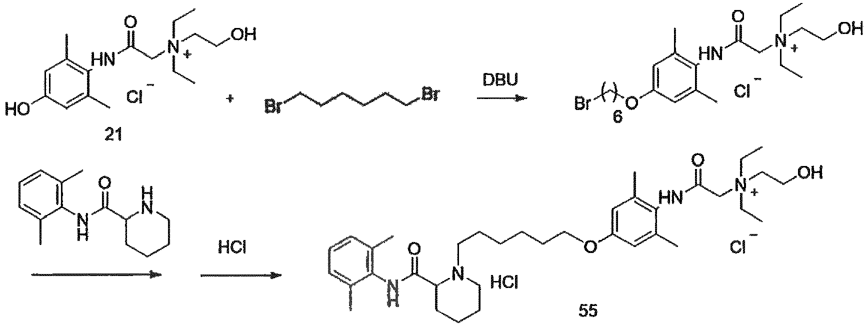

3.30 g of compound 21 was dissolved in 20 mL of DMF, and 3 g of DBU (CAS: 6674-22-2) and 2.44 g of 1,6-dibromohexane were added to the mixture. The obtained mixture was stirred at 80 °C for 6 hours, and added with 1.23 g of 2-pipecolinic acid (CAS: 4043-87-2), and the mixture was stirred to continue to react for 6 hours. 6N hydrochloric acid was added to adjust the pH of the reaction solution to 3. The obtained mixture was evaporated off solvent to dryness under reduced pressure, and the residue was subjected to column chromatography (dichloromethane/methanol=20/1) to obtain 0.95 g of white solid, namely compound 55, with a yield of 13.9%.

1HNMR d-DMSO 400Hz: 1.21-1.42(12H, m), 1.69-1.75(6H, m), 1.93-2.23(14, m), 3.21-3.41(10H, m), 3.96-4.04(4H, m), 4.21(2H, s), 4.49-4.52(1H, m), 6.73(2H, s), 7.03-7.14(3H, m), 10.32(1H, s), 10.72(1H, s).

### Example 56

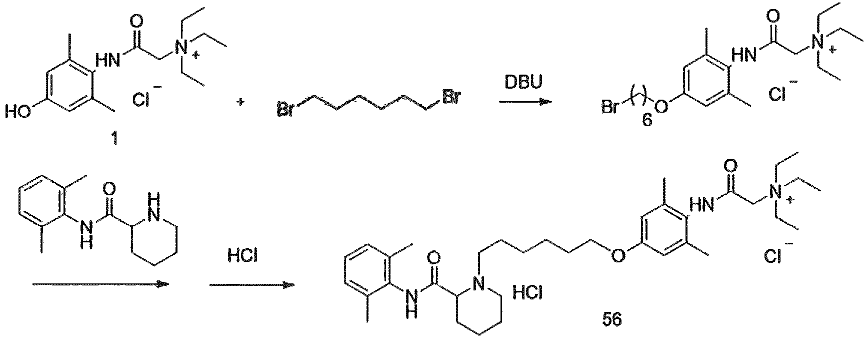

3.15 g of compound 1 was dissolved in 20 mL of DMF, and 3 g of DBU (CAS: 6674-22-2) and 2.44 g of 1,6-dibromohexane were added to the mixture. The obtained mixture was stirred at 80 °C for 6 hours, and added with 1.23 g of 2-pipecolinic acid (CAS: 4043-87-2), and the mixture was stirred to continue to react for 6 hours. 6N hydrochloric acid was added to adjust the pH of the reaction solution to 3. The obtained mixture was evaporated off solvent to dryness under reduced pressure, and the residue was subjected to column chromatography (dichloromethane/methanol=20/1) to obtain 0.75 g of white solid, namely compound 56, with a yield of 9.7%.

1HNMR d-DMSO 400Hz: 1.22-1.32(13H, m), 1.40-1.45(2H, m), 1.69-1.72(6H, m), 1.92-2.23(14, m), 3.21-3.43(10H, m), 3.99-4.09(2H, m), 4.17(2H, s), 4.52-4.56(1H, m), 6.73(2H, s), 7.03-7.13(3H, m), 10.24(1H, s), 10.67 (1H, s).

### Example 57

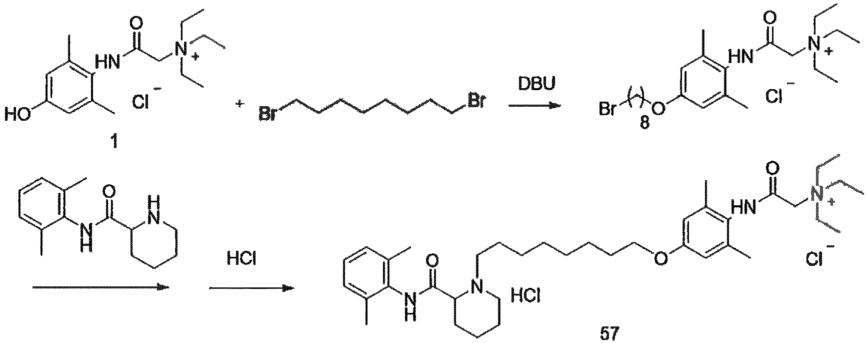

3.15 g of compound 1 was dissolved in 20 mL of DMF, and 3 g of DBU (CAS: 6674-22-2) and 2.71 g of 1,8-dibromooctane were added to the mixture. The obtained mixture was stirred at 80 °C for 6 hours, and added with 1.23 g of 2-pipecolinic acid (CAS: 4043-87-2), and the mixture was stirred to continue to react for 6 hours. 6N hydrochloric acid was added to adjust the pH of the reaction solution to 3. The obtained mixture was evaporated off solvent to dryness under reduced pressure, and the residue was subjected to column chromatography (dichloromethane/methanol=20/1) to obtain 0.67 g of white solid, namely compound 57, with a yield of 11.3%.

1HNMR d-DMSO 400Hz: 1.20-1.32(17H, m), 1.41-1.46(2H, m), 1.71-1.76(6H, m), 1.91-2.21(14, m), 3.19-3.37(10H, m), 4.03-4.10(2H, m), 4.19(2H, s), 4.51-4.54(1H, m), 6.75(2H, s), 7.05-7.14(3H, m), 10.11(1H, s), 10.62 (1H, s).

### Example 58

The compounds of the examples were dissolved in water for injection to a concentration of 0.2-0.5 g/100 mL (0.2-0.5%), or mixed with commercially-available clinically-common local anesthetic drugs to prepare a mixed solution with a specific proportion, and the 0.75% levobupivacaine was used as the positive control compound. The experimental method was as follows: male SD rats weighing 180-300 g were shaved off back hair 1 day before the experiment, and a circular skin area with a diameter of 4 cm was exposed. 0.4 mL of the drug solution was subcutaneously injected to the exposed circular center on the back using a 1 mL syringe to form a skin mound. After 1 minute, the first measurement was performed. Each of 8 random parts around the center of the injection site was stimulated once by acupuncture of 15 g force using a 26G needle (the stimulation site was no more than 1 cm away from the injection center). If the rat showed behaviors such as dodging, shrinking its back or screaming, it was considered that the local anesthetic effect had subsided. If there was no such behavior, it was considered that the local anesthetic effect existed. When 4 or more times of 8 stimulations showed the existence of local anesthetic effect, it was considered that the local anesthetic effect continued to maintain. Except for the first measurement, the measurement was performed every 2 hours within 12 hours after the administration, and every 6 hours after 12 hours after the administration. 8 rats were used for each drug for the experiment. The test results are shown in Table 1.

**Table 1 Anesthesia results of subcutaneous infiltration of the drugs**

| Medicine | Concentration (%) | Duration of local anesthesia (h) |
|---|---|---|
| Levobupivacaine hydrochloride | 0.75 | 4-6 |
| Compound 1 | 0.2 | 6-8 |
| | 0.5 | 10-12 |
| | 0.15+0.35% bupivacaine hydrochloride | 24-30 |
| Compound 3 | 0.2 | 8-14 |
| | 0.5 | 12-18 |
| | 0.15+0.35% bupivacaine hydrochloride | 18-24 |
| Compound 7 | 0.2 | 2-4 |
| | 0.5 | 2-4 |
| | 0.15+0.35% bupivacaine hydrochloride | 4-6 |
| Compound 10 | 0.2 | 18-24 |
| | 0.5 | 24-36 |
| | 0.15+0.35% bupivacaine hydrochloride | 30-36 |
| Compound 12 | 0.2 | 18-24 |
| | 0.5 | 30-48 |
| | 0.15+0.35% bupivacaine hydrochloride | 48-54 |
| Compound 13 | 0.2 | 54-66 |
| | 0.5 | 72-96 |
| | 0.15+0.35% bupivacaine hydrochloride | 72-84 |
| Compound 15 | 0.2 | 24-30 |
| | 0.5 | 42-54 |
| | 0.15+0.35% bupivacaine hydrochloride | 36-42 |
| Compound 17 | 0.2 | 36-48 |
| | 0.5 | 54-60 |
| | 0.15+0.35% bupivacaine hydrochloride | 54-72 |
| Compound 24 | 0.2 | 24-30 |
| | 0.5 | 36-54 |
| | 0.15+0.35% levobupivacaine hydrochloride | 36-42 |
| Compound 26 | 0.2 | 24-36 |
| | 0.5 | 54-66 |
| | 0.15+0.35% levobupivacaine hydrochloride | 42-54 |
| Compound 27 | 0.2 | 18-30 |
| | 0.5 | 36-48 |
| | 0.15+0.5% lidocaine hydrochloride | 36-60 |
| Compound 28 | 0.2 | 36-48 |
| | 0.5 | 54-72 |
| | 0.15+0.35% lidocaine hydrochloride | 54-66 |
| Compound 31 | 0.2 | 24-30 |
| | 0.5 | 36-54 |
| | 0.15+0.35% lidocaine hydrochloride | 36-42 |
| Compound 33 | 0.2 | 36-54 |
| | 0.5 | 60-72 |
| | 0.15+0.15% tetracaine hydrochloride | 66-84 |
| Compound 45 | 0.2 | 24-36 |
| | 0.5 | 42-60 |
| | 0.15+0.15% tetracaine hydrochloride | 36-48 |
| Compound 48 | 0.2 | 36-54 |
| | 0.5 | 60-78 |
| | 0.15+0.15% tetracaine hydrochloride | 66-72 |
| Compound 50 | 0.2 | 18-30 |
| | 0.5 | 24-48 |
| | 0.15+0.15% tetracaine hydrochloride | 42-54 |
| Compound 53 | 0.2 | 24-30 |
| | 0.5 | 48-54 |
| | 0.15+0.15% tetracaine hydrochloride | 36-48 |

The injections of 0.2% and 0.5% of compound 7 were not effective at the first measurement. The other test compounds or compositions produced local anesthetic effects at the first measurement. 0.75% of bupivacaine can only produce local anesthetic effect for 4 to 6 hours, while most of the compounds of the present invention can generally produce local anesthetic effect within the concentration range of 0.2% to 0.5%, in which the longest local anesthetic effect produced can last up to 96 hours. Such local anesthetic effect can resist the stimulation of acupuncture and last for a long time with high intensity. The mixed solution formed by the low concentration of the compounds of the present invention and the low concentration of lidocaine, bupivacaine, levobupivacaine and tetracaine can significantly prolong the duration of local anesthesia, showing strong synergistic effect.

7 days after the end of the experiment, the skin and muscle of the administration site of the experimental animals were taken. Then pathological sections were prepared by HE staining, and the inflammation score of the tissue was observed. The scoring standard was: 0 point (no inflammation); 1 point (local slight inflammation); 2 points (moderate edema, moderate inflammation); 3 points (diffuse edema, severe inflammation). The pathological results show that under the circumstance of the administration of the molecules of the present invention in 0.2% and 0.5%, and the mixed solution formed by the molecules of the present invention with common local anesthetic drugs, the inflammation scores of skin and muscle tissue have no statistical difference with that in the 0.75% levobupivacaine control group, and the damage scores thereof are all 0-1 point, showing that the local safety of the compounds of the present invention is equivalent to that of bupivacaine of a clinical concentration, indicating a good local safety.

### Example 59

The compounds of the examples were dissolved in water for injection to a concentration of 0.5-2.0 g/100 mL (0.5%-2%), or mixed with commercially-available clinically-common local anesthetic drugs to prepare a mixed solution with a specific proportion, and the 0.75% levobupivacaine was used as the positive control compound. The experimental method was as follows: male SD rats weighing 180-300 g were anesthetized with isoflurane, 0.2 mL of the drug-containing solution was injected near the left sciatic nerve of rats, and 8 rats were used for each drug for the experiment. After the rats woke up, the first measurement was performed, and then the measurement was performed every 2 hours. If there was still an anesthetic effect after six measurements, the measurement was performed every 6 hours until the anesthetic effect disappeared. The method for judging the existence of anesthetic effect was as follows. The skin of the sole and lateral surface of the hindlimb of the rat on the side injected with the drug was stimulated using Von Frey filaments (60 g). If the rat showed behaviors such as raising its feet, dodging, it was considered that the local anesthetic effect had disappeared. If there was no such behavior, it was considered that the local anesthetic effect existed. The test results are shown in Table 2.

**Table 2 Results of sciatic nerve blocking experiment in rats**

| Medicine | Concentration (%) | Duration of local anesthesia (h) |
|---|---|---|
| Levobupivacaine hydrochloride | 0.75 | 2-6 |
| Compound 1 | 0.5 | 4-10 |
| | 1.0 | 12-18 |
| | 2.0 | 30-48 |
| | 0.5+0.35% bupivacaine hydrochloride | 18-30 |
| Compound 3 | 0.5 | 10-12 |
| | 1.0 | 18-30 |
| | 2.0 | 54-96 |
| | 0.5+0.35% bupivacaine hydrochloride | 24-36 |
| Compound 7 | 0.5 | <2 |
| | 1.0 | <2 |
| | 2.0 | 2-4 |
| | 0.5+0.35% bupivacaine hydrochloride | 2-4 |
| Compound 10 | 0.5 | 12-24 |
| | 1.0 | 18-30 |
| | 2.0 | 56-96 |
| | 0.5+0.35% bupivacaine hydrochloride | 24-36 |
| Compound 12 | 0.5 | 12-18 |
| | 1 | 24-36 |
| | 2.0 | 72-108 |
| | 0.5+0.35% bupivacaine hydrochloride | 30-48 |
| Compound 13 | 0.5 | 36-42 |
| | 1.0 | 66-78 |
| | 2.0 | 96-144 |
| | 0.5+0.35% bupivacaine hydrochloride | 60-72 |
| Compound 15 | 0.5 | 12-18 |
| | 1.0 | 24-36 |
| | 2.0 | 120-144 |
| | 0.5+0.35% bupivacaine hydrochloride | 30-42 |
| Compound 17 | 0.5 | 24-42 |
| | 1.0 | 42-66 |
| | 2.0 | 96-144 |
| | 0.5+0.35% bupivacaine hydrochloride | 48-66 |
| Compound 24 | 0.5 | 18-30 |
| | 1.0 | 30-48 |
| | 2.0 | 72-160 |
| | 0.5+0.35% levobupivacaine hydrochloride | 36-48 |
| Compound 26 | 0.5 | 24-30 |
| | 1.0 | 48-60 |
| | 2.0 | 96-160 |
| | 0.5+0.35% levobupivacaine hydrochloride | 36-48 |
| Compound 27 | 0.5 | 12-24 |
| | 1.0 | 30-42 |
| | 2.0 | 96-144 |
| | 0.5+0.5% lidocaine hydrochloride | 36-66 |
| Compound 28 | 0.5 | 30-36 |
| | 1.0 | 48-60 |
| | 2.0 | 120-144 |
| | 0.5+0.35% lidocaine hydrochloride | 48-54 |
| Compound 31 | 0.5 | 12-18 |
| | 1.0 | 24-30 |
| | 2.0 | 72-120 |
| | 0.5+0.35% lidocaine hydrochloride | 24-42 |
| Compound 33 | 0.5 | 24-30 |
| | 1.0 | 48-66 |
| | 2.0 | 120-144 |
| | 0.5+0.15% tetracaine hydrochloride | 60-84 |
| Compound 45 | 0.5 | 18-24 |
| | 1.0 | 30-42 |
| | 2.0 | 96-120 |
| | 0.5+0.15% tetracaine hydrochloride | 36-42 |
| Compound 48 | 0.5 | 24-36 |
| | 1.0 | 54-72 |
| | 2.0 | 120-160 |
| | 0.5+0.15% tetracaine hydrochloride | 60-72 |
| Compound 50 | 0.5 | 24-30 |
| | 1.0 | 36-48 |
| | 2.0 | 96-120 |
| | 0.5+0.15% tetracaine hydrochloride | 36-60 |
| Compound 53 | 0.5 | 30-36 |
| | 1.0 | 42-54 |
| | 2.0 | 72-84 |
| | 0.5+0.15% tetracaine hydrochloride | 42-66 |

The drugs to be tested all produced local anesthetic effect at the first measurement. 0.75% bupivacaine can only produce anesthetic effect on the sciatic nerve for 2 to 6 hours, while the longest local anesthetic effect on the sciatic nerve produced by most of the compounds of the present invention within the concentration range of 0.5% to 2.0% can reach 160 hours. The mixed solution formed by the low concentration of the compounds of the present invention and the low concentration of lidocaine, bupivacaine, levobupivacaine and tetracaine can significantly prolong the duration of local anesthesia on the sciatic nerve, showing strong synergistic effect. In addition, 0.75% levobupivacaine hydrochloride not only produced a sensory block on the hindlimb of the rat on the side injected with the drug, but also significantly blocked the motor function of the hindlimb of the rat on this side. The duration of such motor block was basically the same as that of the sensory block. However, none of the compounds disclosed in the present invention have been found to block the motor ability of the hindlimbs of rats, indicating that the compounds of the present invention have the advantage of selective sensory block.

Seven days after the end of the experiment, the sciatic nerve on the side injected with drug of the experimental animals was taken. The pathological sections were prepared by HE staining, and the nerve damage was observed. The scoring standard was: 0 point (slight hyperemia and dilation of epineurial blood vessels, with no inflammatory cell infiltration); 1 point (slight hyperemia and dilation of epineurial blood vessels, with a small amount of inflammatory cell infiltration); 2 points (obvious hyperemia and dilation of epineurial blood vessels, with inflammatory cell infiltration); 3 points (hyperemia and dilation of epineurial blood vessels, with inflammatory cell infiltration in spinal cord parenchyma). The pathological results show that under the circumstance of the administration of the molecules of the present invention in 0.5% and 1.0%, and the mixed solution with a low concentration formed by the molecules of the present invention with common local anesthetic drugs, the inflammation of the epineurium has no statistical difference with that of the 0.75% levobupivacaine control group, and the injury scores thereof are all 0 point. In addition, no demyelination was found in all rat sciatic nerve samples under electron microscopy. The above results show that the local safety of the compounds of the present invention is equivalent to that of bupivacaine of a clinical concentration, indicating a good local safety.

### Example 60

0.2% aqueous solution of the compounds of the present invention and 0.75% levobupivacaine solution were injected into the rats through tail vein in 0.4 mL, and the mortality rate was observed. Each drug was injected into 10 rats (half male and half female, weighing 290-310). The results are shown in Table 3.

**Table 3 Mortality rate after the injection of drugs through tail vein**

| Medicine | Mortality rate % |
|---|---|
| Levobupivacaine hydrochloride | 70 |
| Compound 1 | 0 |
| Compound 3 | 0 |
| Compound 10 | 0 |
| Compound 12 | 0 |
| Compound 13 | 0 |
| Compound 15 | 0 |
| Compound 17 | 0 |
| Compound 24 | 0 |
| Compound 26 | 0 |
| Compound 27 | 0 |
| Compound 28 | 0 |
| Compound 31 | 0 |
| Compound 33 | 0 |
| Compound 45 | 0 |
| Compound 48 | 0 |
| Compound 50 | 0 |
| Compound 53 | 0 |

In Example 51, at a concentration of 0.2%, the compounds of the present invention can produce a local infiltration anesthetic effect for 24-66 hours with an injection volume of 0.4 mL. If the compound of this dose was all mistakenly injected into the blood, it was known from this experiment that the rats would not die. However, 0.4 mL of 0.75% levobupivacaine can only produce local infiltration anesthetic effect for 4 to 6 hours. If levobupivacaine of this dose was all mistakenly injected into the blood, 70% of the rats would die. It can be seen that the compounds of the present invention have high titer and good safety, and the overall safety thereof at an effective dose is much higher than that of the control drug levobupivacaine.

## Claims

1. A compound, or pharmaceutically acceptable salt of the formula (I): 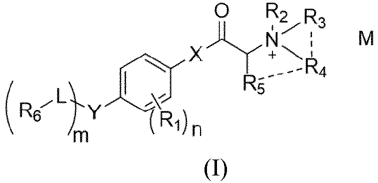 wherein:
R₁ is selected from the group consisting of C1-5 alkyl, halogen;
R₂ is selected from the group consisting of C1-4 alkyl;
R₃ is selected from the group consisting of C1-4 alkyl;
R₄ is selected from the group consisting of C1-4 alkyl;
R₅ is H;
X is HN;
Y is O;
L is carbonyl;
m=1;
n is an integer from 0 to 4; and
R₆ is C1-12 alkyl;
any carbon atom in the skeleton of R₁-R₇ can be replaced by N atom or O atom;
the skeleton of R₁-R₇ can be substituted by one or more hydroxyl; and
M is a pharmaceutically acceptable anion, such as chloride ion, bromide ion, sulfate ion, acetate ion, and sulfonate ion;
or
a compound, or pharmaceutically acceptable salt of the formula (I): 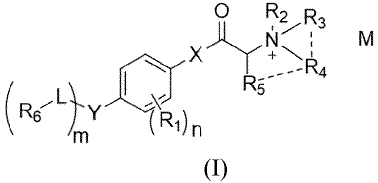
wherein:
R₁ is selected from the group consisting of C1-2 alkyl;
R₂ is selected from the group consisting of C1-3 alkyl;
R₃ is selected from the group consisting of C1-3 alkyl;
R₄ is selected from the group consisting of C1-3 alkyl;
R₅ is H;
X is HN;
Y is O;
L is carbonyl;
m=1;
n is an integer from 0 to 4; and
R₆ is the following specified structure: 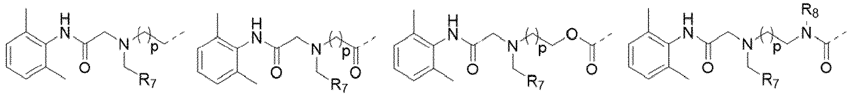 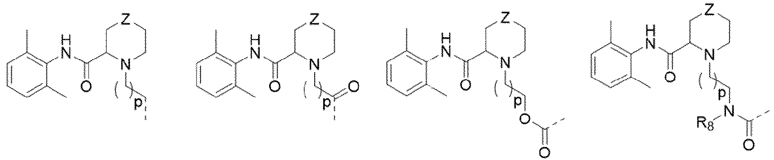
in the above specified structure, p is an integer from 1-10, R₇ is C1-4 alkyl, R₈ is H or C1-2 alkyl, Z is methylene or O;
any carbon atom in the skeleton of R₁-R₇ can be replaced by O atom or N atom;
the skeleton of R₁-R₇ can be substituted by one or more hydroxyl; and
M is a pharmaceutically acceptable anion, such as chloride ion, bromide ion, sulfate ion, acetate ion, and sulfonate ion.

2. A compound or a pharmaceutically acceptable salt thereof, wherein the compound is selected from the following specific compounds:

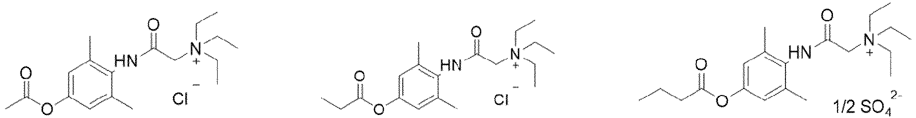

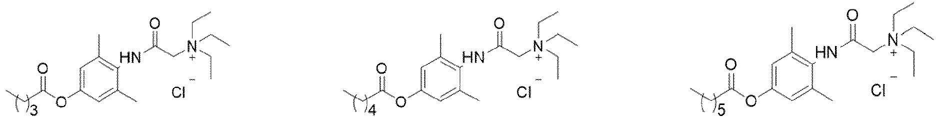

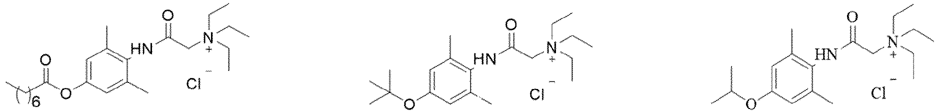

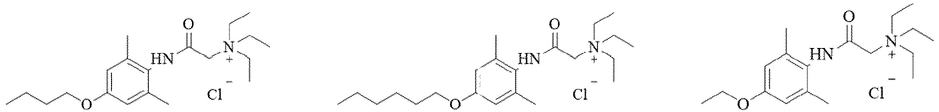

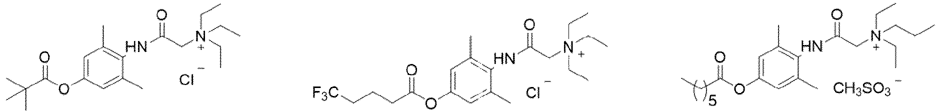

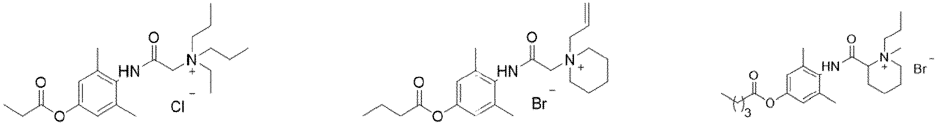

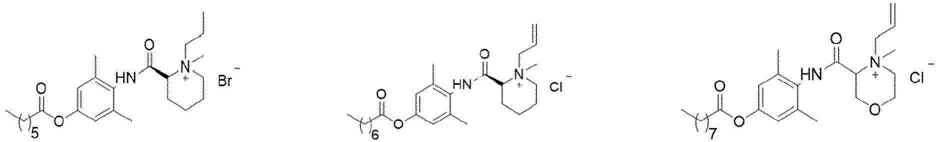

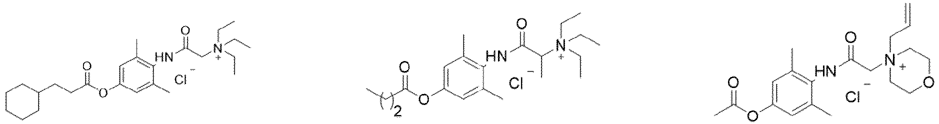

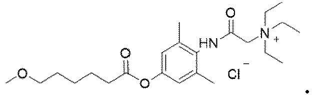

3. A compound or a pharmaceutically acceptable salt thereof, wherein the compound is selected from the following specific compounds:

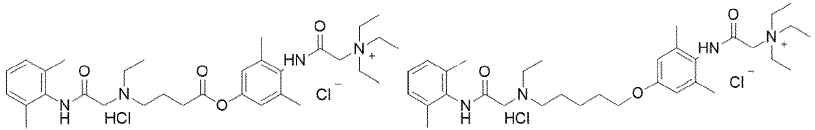

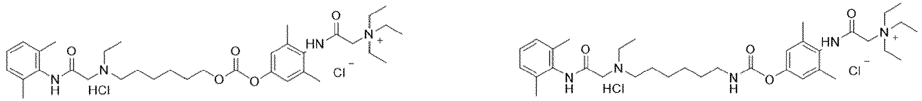

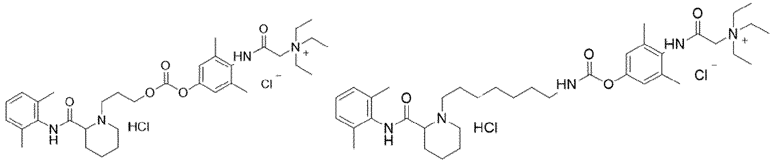

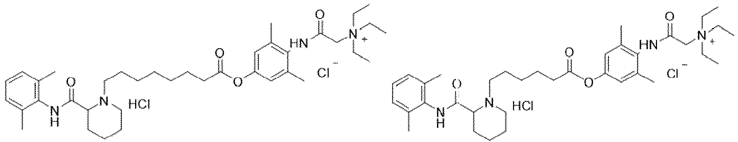

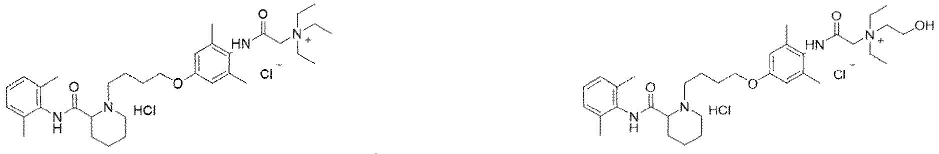

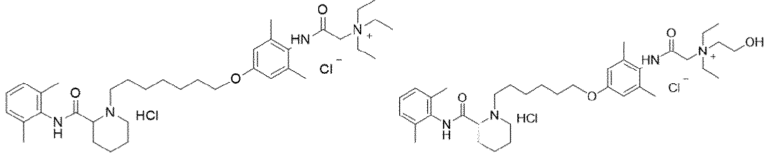

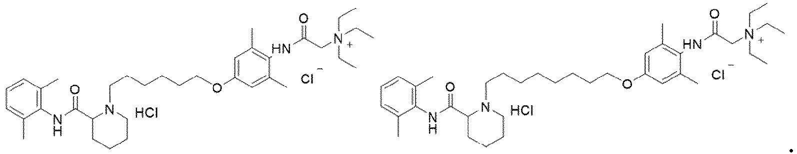

4. The compound, or pharmaceutically acceptable salt according to any one of claims 1 to 3 for use as a local anesthetic, analgesic or antipruritic drug, which locally produces a fast, lasting and safe nerve blocking effect.

5. A pharmaceutical composition, comprising the compound according to any one of claims 1 to 3 and lidocaine or lidocaine salt.

6. A pharmaceutical composition, comprising the compound according to any one of claims 1 to 3 and bupivacaine, levobupivacaine, tetracaine or salt thereof.

7. A pharmaceutical composition, comprising the compound according to any one of claims 1 to 3 and ion channel agonists such as tetrodotoxin and capsaicin.

8. The pharmaceutical composition according to any one of claims 5 to 7 for use as a local anesthetic, analgesic or antipruritic drug, which locally produces a fast, lasting and safe nerve blocking effect.

9. A sustained-release preparation, comprising the compound, or pharmaceutically acceptable salt according to any one of claims 1 to 3 and sustained-release materials.

10. The sustained-release preparation according to claim 9 for use as a long-acting local anesthetic, analgesic or antipruritic drug.

## Patentansprüche

1. Verbindung oder pharmazeutisch verträgliches Salz der Formel (I): 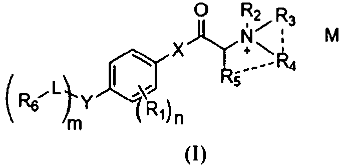 wobei:
R₁ ausgewählt ist aus der Gruppe bestehend aus C1-5-Alkyl, Halogen;
R₂ ausgewählt ist aus der Gruppe bestehend aus C1-4-Alkyl;
R₃ ausgewählt ist aus der Gruppe bestehend aus C1-4-Alkyl;
R₄ ausgewählt ist aus der Gruppe bestehend aus C1-4-Alkyl;
R₅ H ist;
X HN ist;
Y O ist;
L Carbonyl ist;
m = 1;
n eine ganze Zahl zwischen 0 und 4 ist; und
R₆ C1-12-Alkyl ist;
jedes Kohlenstoffatom im Gerüst von R₁-R₇ durch ein N-Atom oder ein O-Atom ersetzt sein kann;
das Gerüst von R₁-R₇ durch eine oder mehrere Hydroxylgruppen substituiert sein kann; und
M ein pharmazeutisch akzeptables Anion ist, wie beispielsweise ein Chloridion, Bromidion, Sulfation, Acetation und Sulfonation;
oder
eine Verbindung oder ein pharmazeutisch verträgliches Salz der Formel (I): 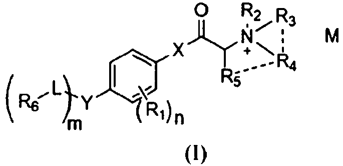
wobei:
R₁ ausgewählt ist aus der Gruppe bestehend aus C1-2-Alkyl;
R₂ ausgewählt ist aus der Gruppe bestehend aus C1-3-Alkyl;
R₃ ausgewählt ist aus der Gruppe bestehend aus C1-3-Alkyl;
R₄ ausgewählt ist aus der Gruppe bestehend aus C1-3-Alkyl;
R₅ H ist;
X HN ist;
Y O ist;
L Carbonyl ist;
m = 1;
n eine ganze Zahl zwischen 0 und 4 ist; und
R₆ die folgende spezifizierte Struktur ist: 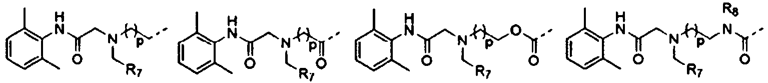 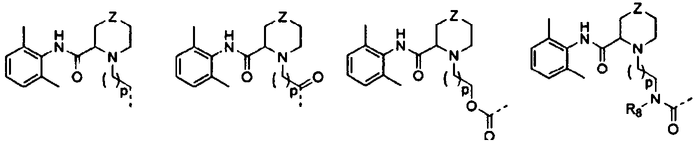
in der oben spezifizierten Struktur p eine ganze Zahl von 1 bis 10 ist, R₇ C1-4-Alkyl ist, R₈ H oder C1-2-Alkyl ist, Z Methylen oder O ist;
jedes Kohlenstoffatom im Gerüst von R₁-R₇ durch ein O-Atom oder ein N-Atom ersetzt sein kann;
das Gerüst von R₁-R₇ durch eine oder mehrere Hydroxylgruppen substituiert sein kann; und
M ein pharmazeutisch akzeptables Anion ist, wie beispielsweise ein Chloridion, Bromidion, Sulfation, Acetation und Sulfonation.

2. Verbindung oder pharmazeutisch verträgliches Salz davon, wobei die Verbindung aus den folgenden spezifischen Verbindungen ausgewählt ist:

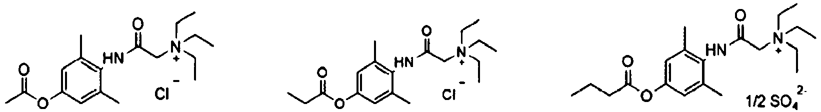

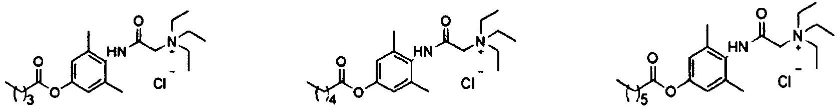

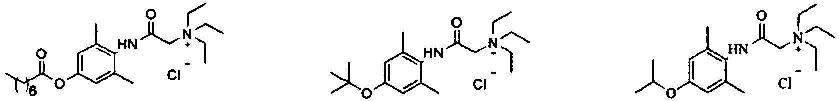

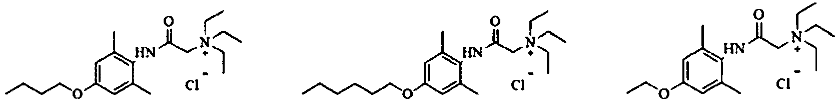

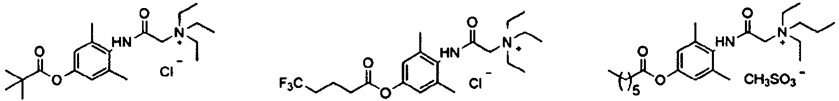

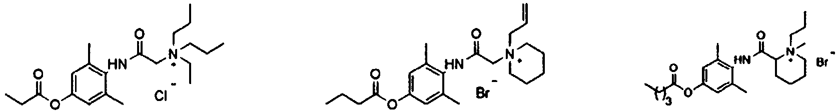

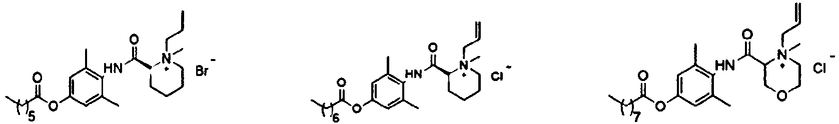

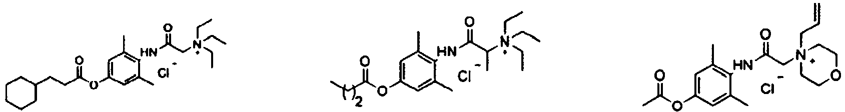

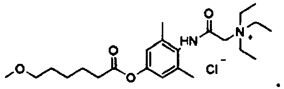

3. Verbindung oder pharmazeutisch verträgliches Salz davon, wobei die Verbindung aus den folgenden spezifischen Verbindungen ausgewählt ist:

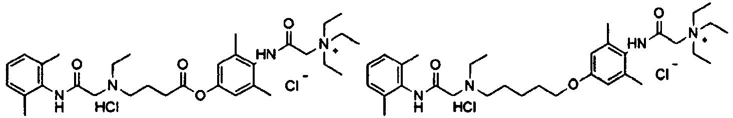

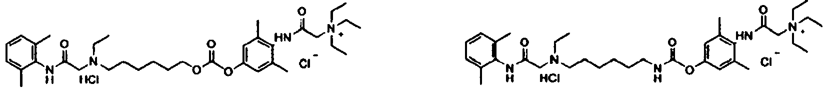

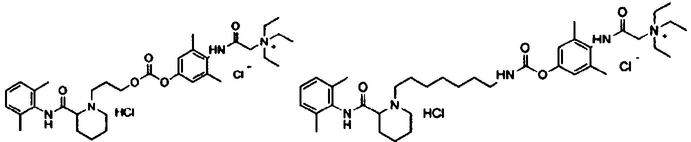

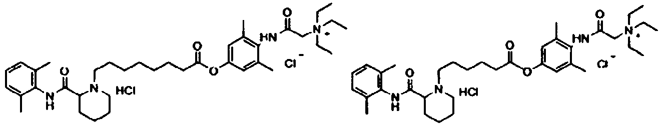

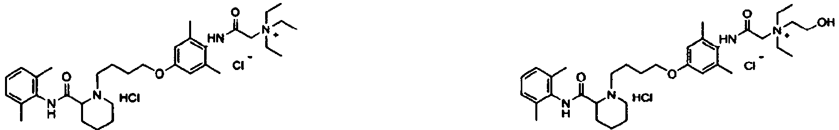

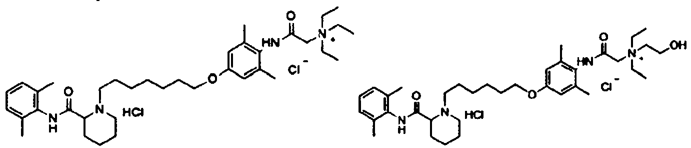

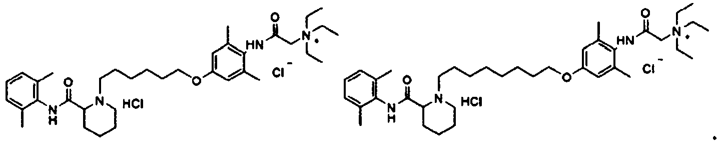

4. Verbindung oder pharmazeutisch verträgliches Salz nach einem der Ansprüche 1 bis 3 zur Verwendung als ein Lokalanästhetikum, Analgetikum oder Antipruriginosum, das lokal eine schnelle, anhaltende und sichere Nervenblockade bewirkt.

5. Pharmazeutische Zusammensetzung, umfassend die Verbindung nach einem der Ansprüche 1 bis 3 und Lidocain oder Lidocainsalz.

6. Pharmazeutische Zusammensetzung, umfassend die Verbindung nach einem der Ansprüche 1 bis 3 und Bupivacain, Levobupivacain, Tetracain oder ein Salz davon.

7. Pharmazeutische Zusammensetzung, umfassend die Verbindung nach einem der Ansprüche 1 bis 3 und Ionenkanalagonisten wie Tetrodotoxin und Capsaicin.

8. Pharmazeutische Zusammensetzung nach einem der Ansprüche 5 bis 7 zur Verwendung als ein Lokalanästhetikum, Analgetikum oder Antipruriginosum, das lokal eine schnelle, anhaltende und sichere Nervenblockade bewirkt.

9. Präparat mit verzögerter Freisetzung, umfassend die Verbindung oder ein pharmazeutisch verträgliches Salz nach einem der Ansprüche 1 bis 3 und Materialien mit verzögerter Freisetzung.

10. Präparat mit verzögerter Freisetzung nach Anspruch 9 zur Verwendung als ein langwirksames Lokalanästhetikum, Analgetikum oder Antipruriginosum.

## Revendications

1. Composé, ou sel pharmaceutiquement acceptable selon la formule (1) : 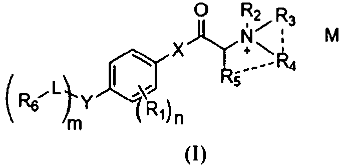 dans lesquels
R₁ est sélectionné dans le groupe constitué d'alkyle en C1-5, halogène ;
R₂ est sélectionné dans le groupe constitué d'alkyle en C1-4 ;
R₃ est sélectionné dans le groupe constitué d'alkyle en C1-4 ;
R₄ est sélectionné dans le groupe constitué d'alkyle en C1-4 ;
R₅ est H ;
X est HN ;
Y est O ;
L est carbonyle ;
m = 1 ;
n est un nombre entier de 0 à 4 ; et
R₆ est un alkyle en C1-12 ;
tout atome de carbone dans le squelette de R₁-R₇ peut être remplacé par un atome d'azote ou un atome d'oxygène ;
le squelette de R₁-R₇ peut être substitué par un ou plusieurs groupes hydroxyle ; et M est un anion pharmaceutiquement acceptable, tel que ion chlorure, ion bromure, ion sulfate, ion acétate, et ion sulfonate ;
ou
un composé, ou sel pharmaceutiquement acceptable selon la formule (I) : 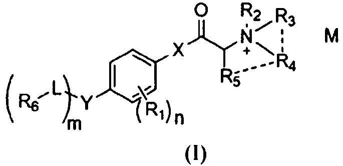
dans lesquels
R₁ est sélectionné dans le groupe constitué d'alkyle en C1-2 ;
R₂ est sélectionné dans le groupe constitué d'alkyle en C1-3 ;
R₃ est sélectionné dans le groupe constitué d'alkyle en C1-3 ;
R₄ est sélectionné dans le groupe constitué d'alkyle en C1-3 ;
R₅ est H ;
X est HN ;
Y est O ;
L est carbonyle ;
m = 1 ;
n est un nombre entier de 0 à 4 ; et
R₆ est la structure spécifiée suivante : 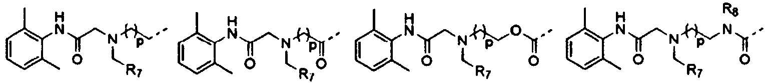 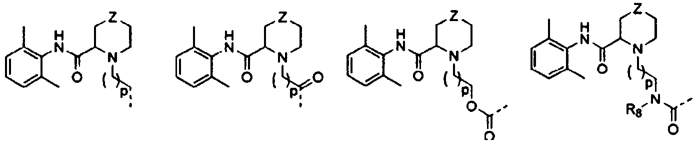
dans la structure spécifiée ci-dessus, p est un nombre entier de 1-10, R₇ est un alkyle en C1-4, R₈ est H ou un alkyle en C1-2, Z est le méthylène ou O ;
tout atome de carbone dans le squelette de R₁-R₇ peut être remplacé par un atome d'oxygène ou un atome d'azote ;
le squelette de R₁-R₇ peut être substitué par un ou plusieurs groupes hydroxyle ; et M est un anion pharmaceutiquement acceptable, tel que ion chlorure, ion bromure, ion sulfate, ion acétate, et ion sulfonate.

2. Composé ou sel pharmaceutiquement acceptable de celui-ci, dans lequel le composé est sélectionné parmi les composés spécifiques suivants :

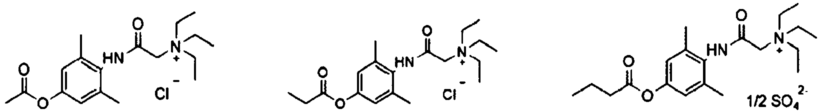

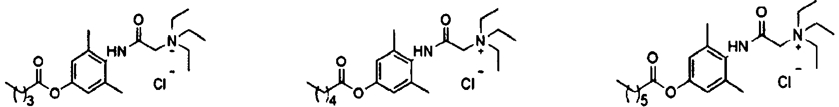

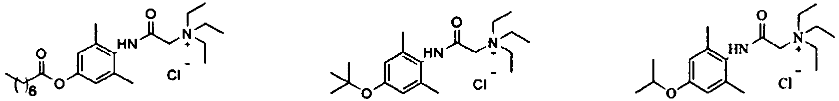

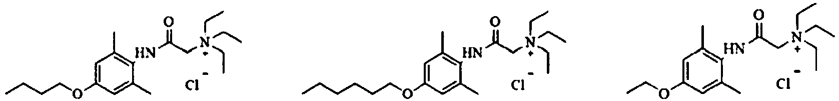

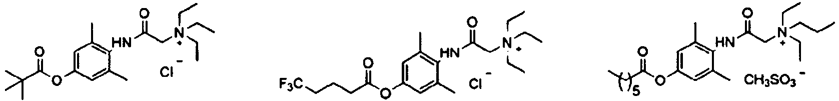

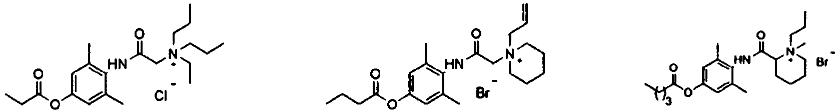

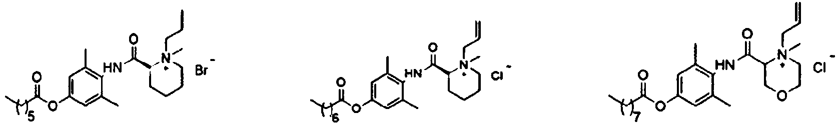

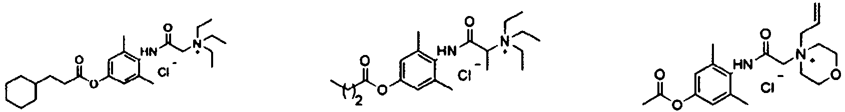

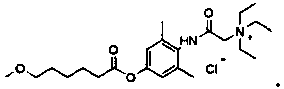

3. Composé ou sel pharmaceutiquement acceptable de celui-ci, dans lequel le composé est sélectionné parmi les composés spécifiques suivants :

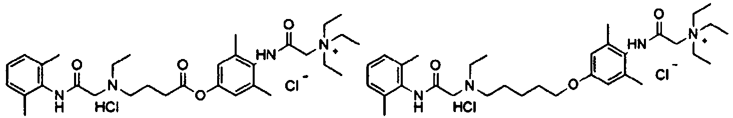

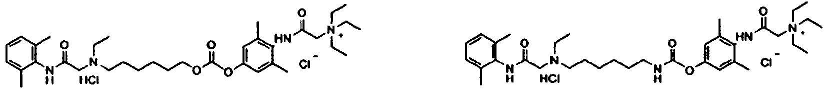

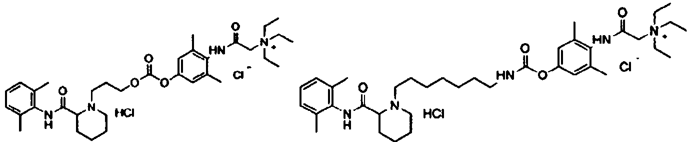

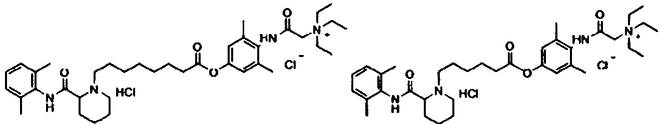

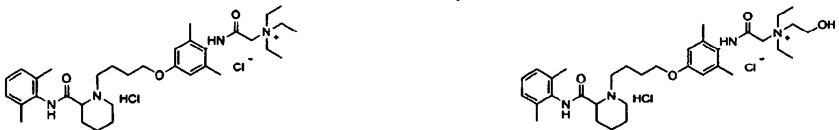

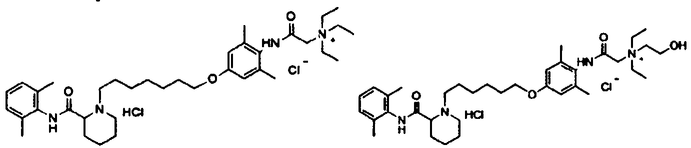

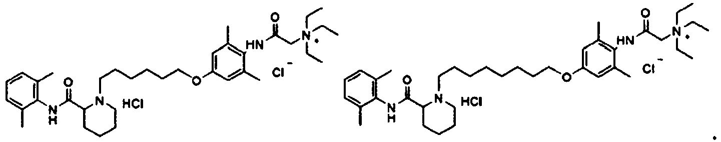

4. Composé, ou sel pharmaceutiquement acceptable selon l'une quelconque des revendications 1 à 3 pour une utilisation en tant que médicament local anesthésique, analgésique ou antipruritique, qui produit localement un effet de blocage nerveux rapide, durable et sûr.

5. Composition pharmaceutique, comprenant le composé selon l'une quelconque des revendications 1 à 3 et la lidocaïne ou un sel de lidocaïne.

6. Composition pharmaceutique, comprenant le composé selon l'une quelconque des revendications 1 à 3 et la bupivacaïne, la lévobupivacaïne, la tétracaïne ou un sel de celles-ci.

7. Composition pharmaceutique, comprenant le composé selon l'une quelconque des revendications 1 à 3 et des agonistes des canaux ioniques tels que la tétrodotoxine et la capsaïcine.

8. Composition pharmaceutique selon l'une quelconque des revendications 5 à 7 pour une utilisation en tant que médicament local anesthésique, analgésique ou antipruritique, qui produit localement un effet de blocage nerveux rapide, durable et sûr.

9. Préparation à libération prolongée, comprenant le composé, ou sel pharmaceutiquement acceptable selon l'une quelconque des revendications 1 à 3 et des matériaux à libération prolongée.

10. Préparation à libération prolongée selon la revendication 9 pour une utilisation en tant que médicament local anesthésique, analgésique ou antipruritique à longue durée d'action.
